(19) Europäisches Patentamt
European Patent Office
Office européen des brevets

(11) **EP 3 659 641 B1**

(12) **EUROPEAN PATENT SPECIFICATION**

(45) Date of publication and mention
of the grant of the patent:
**12.07.2023 Bulletin 2023/28**

(21) Application number: **18837527.3**

(22) Date of filing: **25.07.2018**

(51) International Patent Classification (IPC):
*A61M 1/16* (2006.01)    *A61M 1/34* (2006.01)
*A61M 1/36* (2006.01)

(52) Cooperative Patent Classification (CPC):
**A61M 1/3403; A61M 1/16;** A61M 1/3626

(86) International application number:
**PCT/JP2018/027831**

(87) International publication number:
**WO 2019/022113 (31.01.2019 Gazette 2019/05)**

(54) **BLOOD PURIFYING DEVICE, METHOD FOR OBTAINING TRANSMEMBRANE PRESSURE DIFFERENCE ACROSS BLOOD PURIFICATION MEMBRANE, AND METHOD, DEVICE AND PROGRAM FOR DETERMINING SAME**

BLUTREINIGUNGSVORRICHTUNG, VERFAHREN ZUM ERHALT DER TRANSMEMBRANDRUCKDIFFERENZ BEI EINER BLUTREINIGUNGSMEMBRAN SOWIE VERFAHREN, VORRICHTUNG UND PROGRAMM ZUR BESTIMMUNG DERSELBEN

DISPOSITIF D'ÉPURATION DU SANG, PROCÉDÉ D'OBTENTION D'UNE DIFFÉRENCE DE PRESSION TRANSMEMBRANAIRE À TRAVERS UNE MEMBRANE D'ÉPURATION DU SANG, ET PROCÉDÉ, DISPOSITIF ET PROGRAMME DE DÉTERMINATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2017 JP 2017145863**
**20.04.2018 JP 2018081667**

(43) Date of publication of application:
**03.06.2020 Bulletin 2020/23**

(73) Proprietor: **Asahi Kasei Medical Co., Ltd.**
**Tokyo 100-0006 (JP)**

(72) Inventors:
• **SASAKI,Kohsuke**
**Tokyo 101-8101 (JP)**

• **MASAOKA, Katsunori**
**Hirosima-shi**
**Hiroshima 730-0812 (JP)**
• **TAOKA, Masahiro**
**Nagoya-shi**
**Aichi 454-0933 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2013/141309     WO-A1-2013/141309**
**JP-A- 2001 112 863     JP-A- 2001 112 863**

## Description

## Technical Field

The present invention relates to a blood purifying device, a method, a device, and a program for obtaining a transmembrane pressure difference across a blood purification membrane, and a method, a device, and a program for determining the transmembrane pressure difference across the blood purification membrane.

## Background Art

Blood purification treatment such as hemodialysis therapy, leukocyte removal therapy, or plasma exchange therapy is performed using a blood purifying device. For example, a blood purifying device for performing hemodiafiltration (HDF) includes a blood purifier including a blood purification membrane such as a hollow fiber membrane, a blood circuit which delivers blood from a patient to the blood purifier and returns the blood to the patient, a dialysate circuit which supplies/discharges a dialysate to/from the blood purifier, a replacement fluid means that supplies a replacement fluid to the blood circuit, and the like. When hemodiafiltration is performed, blood is supplied from the patient to a primary side of the blood purification membrane through the blood circuit, while the dialysate is supplied to a secondary side of the blood purification membrane through the dialysate circuit. By diffusion and filtration, a disease agent in the blood on the primary side of the blood purification membrane is discharged to the secondary side of the blood purification membrane to purify the blood (see Patent Document 1).

However, as the blood purification treatment proceeds in a blood purifying device as described above, a transmembrane pressure difference (TMP) across the blood purification membrane is increased by clogging of the blood purification membrane or the like. When the TMP is increased, a large amount of albumin leaks out of the blood to the secondary side (discharge side) of the blood purification membrane. Since albumin is an essential protein, it is not preferable that the large amount of albumin leaks out of the blood.

JP 2001-112863 describes controlling a transmembrane pressure difference within a predetermined time in order to suppress the loss of albumin.

WO 2013/141309 describes using a preset regression equation to define a transmembrane pressure difference at which target albumin leakage quantity is met, said transmembrane pressure difference being a blood purification condition.

Citation List

Patent Document

Patent Document 1: Japanese Patent No. 6,070,348

Summary

Technical Problem

To prevent this, it can be considered to perform, e.g., adjustment of a flow rate of the replacement fluid (flow rate of a replacement fluid pump) supplied to the blood circuit or the like and thus adjust a pressure on a blood circuit side and a pressure on a dialysate circuit side so as to hold the TMP constant.

However, when the TMP is held constant as described above, it is possible to inhibit leakage of a large amount of albumin, but it is impossible to control an amount of albumin leakage. In dialytic therapy or the like, it is requested to control the amount of albumin leakage and, for example, it is desired to control the amount of albumin leakage per therapy session to 4 g or less.

The present application has been filed in view of such circumstances, and an object of the present application is to provide a blood purifying device capable of controlling an amount of leakage of a specified solute in blood such as albumin when the blood is purified by a blood purification membrane, while inhibiting the leakage of the solute, a method, a device, and a program for obtaining a transmembrane pressure difference across the blood purification membrane, and a method, a device, and a program for determining the transmembrane pressure difference across the blood purification membrane.

Solution to Problem

As a result of conducting vigorous study, the present inventors have found that, by controlling a transmembrane pressure difference on a basis of a function Fn showing a relationship between a transmembrane pressure difference P(Tn) and a solute leakage concentration N(Tn) of a specified solute in blood at each of a plurality of predetermined times Tn after initiation of blood purification, it is possible to achieve the object described above and have completed the present invention.

Specifically, the present invention is directed to the subject-matter reflected in the appendant claims.

Advantageous Effects of the Invention

According to the present invention, it is possible to control an amount of leakage of a specified solute in blood when the blood is purified by a blood purification membrane, while inhibiting the leakage of the solute.

**Brief Description of Drawings**

Fig. 1 is an illustrative view showing a schematic configuration of a blood purifying device.

Fig. 2 is a graph showing an example of a function Fn.

Fig. 3 is a block diagram of a control means.

Fig. 4 is a block diagram of a function acquisition means.

Fig. 5 is a graph for illustrating an example of a method for acquiring a function Fn.

Fig. 6 is a graph for illustrating an example of control of a transmembrane pressure difference.

Fig. 7 is a graph showing an example of a target solute leakage concentration during blood purification.

Fig. 8 is a graph showing an example of a set transmembrane pressure difference during the blood purification.

Fig. 9 is a graph for illustrating an example of another method for acquiring the function Fn.

Fig. 10 is a graph showing another example of the set transmembrane pressure difference during the blood purification.

Fig. 11 is a graph showing a correlation between an average value Pi of the transmembrane pressure differences and a solute leakage amount Ai during 240 minutes after initiation of the blood purification.

Fig. 12 is a graph showing a correlation between a transmembrane pressure difference Pj of the function Fn and a solute leakage concentration Nj at S = 30 minutes as a time 30 minutes after the initiation of blood purification.

Fig. 13 is a block diagram showing an estimation means and a display means.

Fig. 14 illustrates an example of a data map representing a solute leakage concentration Nm(Tn) in a combination of each of times Tn and a transmembrane pressure difference Pm(Tn).

Fig. 15 illustrates an example of display representing an amount of albumin leakage and a set transmembrane pressure difference at each of the times during the blood purification.

Fig. 16 is a graph representing a difference between a target solute leakage concentration Na(Tn) and an actually measured value RNa(Tn) of the solute leakage concentration.

Fig. 17 is a graph representing an example of a case where a supply flow rate of a replacement fluid reaches an upper limit.

Fig. 18 is a graph representing an example of a case where the set transmembrane pressure difference is corrected so as to prevent the supply flow rate of the replacement fluid from reaching the upper limit.

**Description of Embodiments**

Referring to the drawings, the following will describe preferred embodiments of the present invention. Note that the same elements are denoted by the same reference signs, and a repetitive explanation thereof is omitted. Positional relationships, such as a vertical positional relationship and a lateral relationship, are based on those shown in the drawings unless particularly described otherwise. In addition, dimensional ratios in the drawings are not limited to those illustrated. The following embodiments are exemplary for the purpose of describing the present invention.

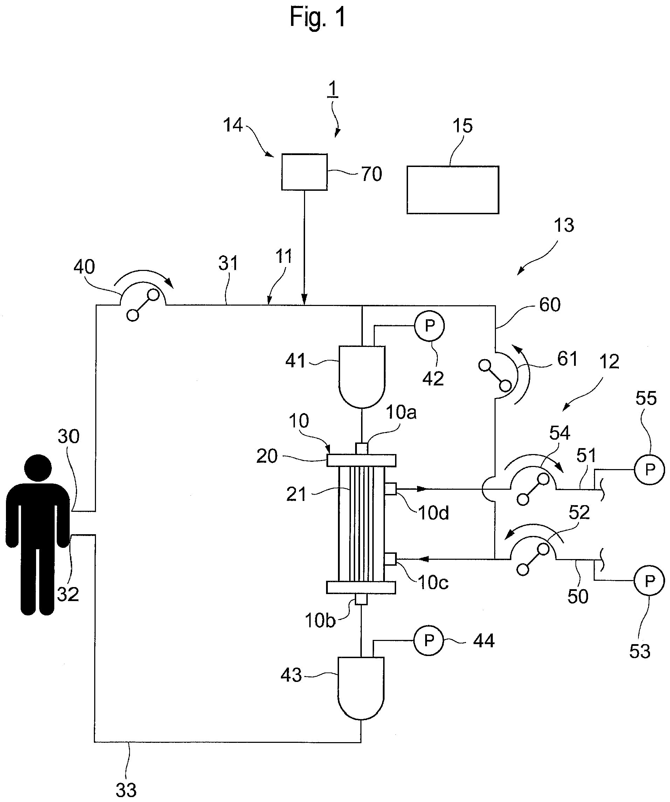

Fig. 1 is an illustrative view illustrating a schematic configuration of a blood purifying device 1 according to the present embodiment. The blood purifying device 1 of the present embodiment is intended to perform, e.g., hemodiafiltration (HDF).

For example, the blood purifying device 1 includes a blood purifier 10, a blood circuit 11, a dialysate circuit 12, a replacement fluid circuit 13, an anticoagulant supply circuit 14, a control means 15, and the like.

The blood purifier 10 has a cylindrical main body 20 and internally includes a blood purification membrane 21 made of a hollow fiber membrane or the like. The blood purifier 10 has, in the main body 20, fluid vent holes 10a and 10b connecting to a primary side (blood side) of the blood purification membrane 21 and fluid vent holes 10c and 10d connecting to a secondary side (dialysate side) of the blood purification membrane 21.

For example, the blood circuit 11 has a blood collection circuit 31 connecting a blood collection portion 30 and the fluid vent hole 10a of the blood purifier 10 and a blood return circuit 33 connecting the fluid vent hole 10b of the blood purifier 10 and a blood return portion 32. The blood circuit 11 is formed of a soft tube.

For example, in the blood collection circuit 31, a blood pump 40, a drip chamber 41, a pressure sensor 42, and the like are provided. As the blood pump 40, e.g., a tube pump is used.

For example, in the blood return circuit 33, a drip chamber 43, a pressure sensor 44, and the like are provided.

For example, the dialysate circuit 12 has a dialysate supply circuit 50 leading from, e.g., a dialysate supply source not shown to the fluid vent hole 10c of the blood purifier 10 and a dialysate discharge circuit 51 leading from the fluid vent hole 10d of the blood purifier 10 to the outside of the device. For example, in the dialysate supply circuit 50, a dialysate supply pump 52, a pressure sensor 53, and the like are provided. For example, in the dialysate discharge circuit 51, a dialysate discharge pump 54, a pressure sensor 55, and the like are provided.

For example, the replacement fluid circuit 13 includes a replacement fluid line 60 which connects the dialysate supply circuit 50 and the blood collection circuit 31 and a replacement fluid pump 61 which supplies the dialysate from the dialysate supply circuit 50 as a replacement fluid to the blood collection circuit 31.

The anticoagulant supply circuit 14 has a fluid reservoir 70 in which, e.g., an anticoagulant is stored and can supply a predetermined amount of the anticoagulant from the fluid reservoir 70 to the blood collection circuit 31.

For example, the control means 15 is a microcomputer including a CPU, a memory, and the like. The control means 15 controls respective operations of such devices as, e.g., the blood pump 40, the dialysate supply pump 52, the dialysate discharge pump 53, and the replacement fluid pump 61 to control a blood-side flow rate, a dialysate-side flow rate, a supply flow rate of the replacement fluid, and the like and thus allow blood purification treatment to be executed. For example, the control means 15 executes a program stored in advance in the memory to allow the blood purification treatment to be accomplished.

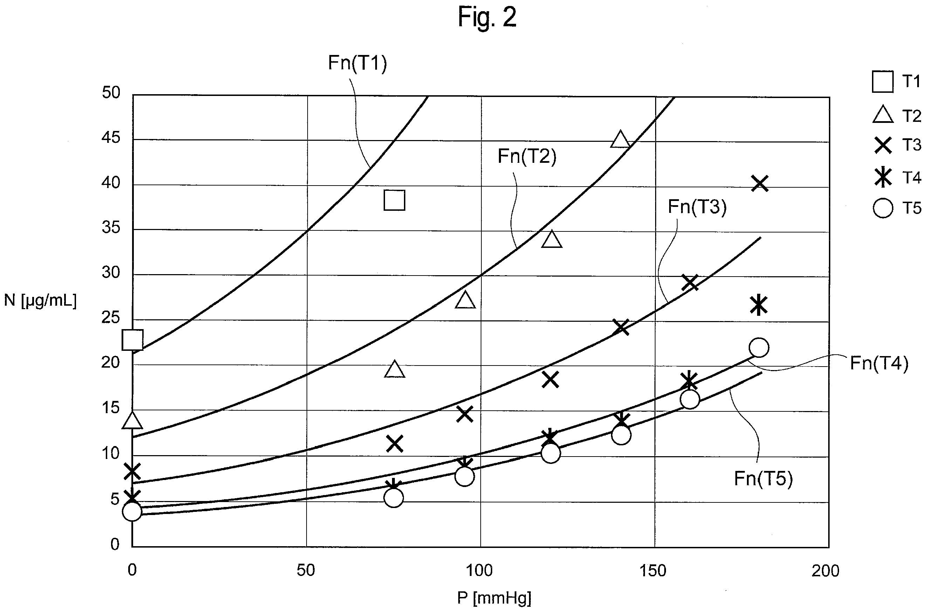

For example, the control means 15 controls a transmembrane pressure difference (TMP(pressure difference between the primary side and the secondary side of the blood purification membrane 21)) across the blood purification membrane 21 of the blood purifier 10. The control means 15 controls, on the basis of a function Fn given by a numerical expression (1) and showing a relationship between a transmembrane pressure difference $P(Tn)$ at each of one or more predetermined times Tn after initiation of blood purification and a solute leakage concentration $N(Tn)$ (N is a concentration at the secondary-side fluid vent hole 10d of the blood purification membrane 21) of albumin as a predetermined solute in blood, which is acquired in advance and as shown in Fig. 2, a transmembrane pressure difference at each of the times Tn during the blood purification:

$$N(Tn) = Fn(P(Tn), Tn) \quad \text{(n is an integer of not less than 1) ... (1).}$$

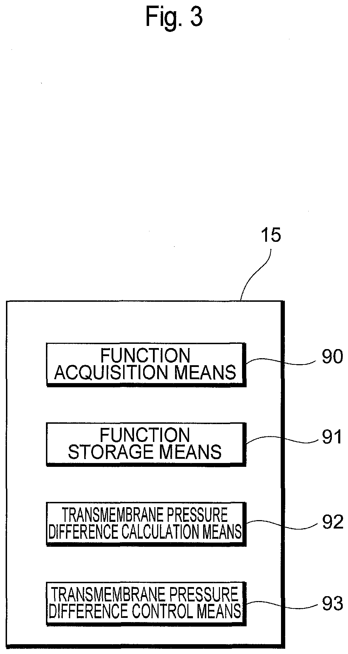

As shown in Fig. 3, the control means 15 has a function acquisition means 90 which acquires the function Fn, a function storage means 91 which stores the function Fn, a transmembrane pressure difference calculation means 92 which obtains, on the basis of the function Fn, a transmembrane pressure difference $Pa(Tn)$ corresponding to a target solute leakage concentration $Na(Tn)$ at each of the times Tn during the blood purification, and a transmembrane pressure difference control means 93 which controls, on the basis of the transmembrane pressure difference $Pa(Tn)$, the transmembrane pressure difference at each of the times Tn during the blood purification.

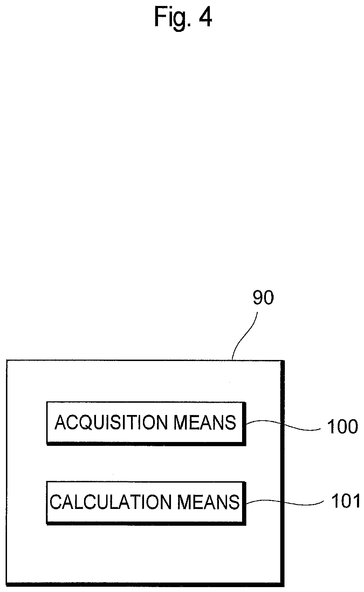

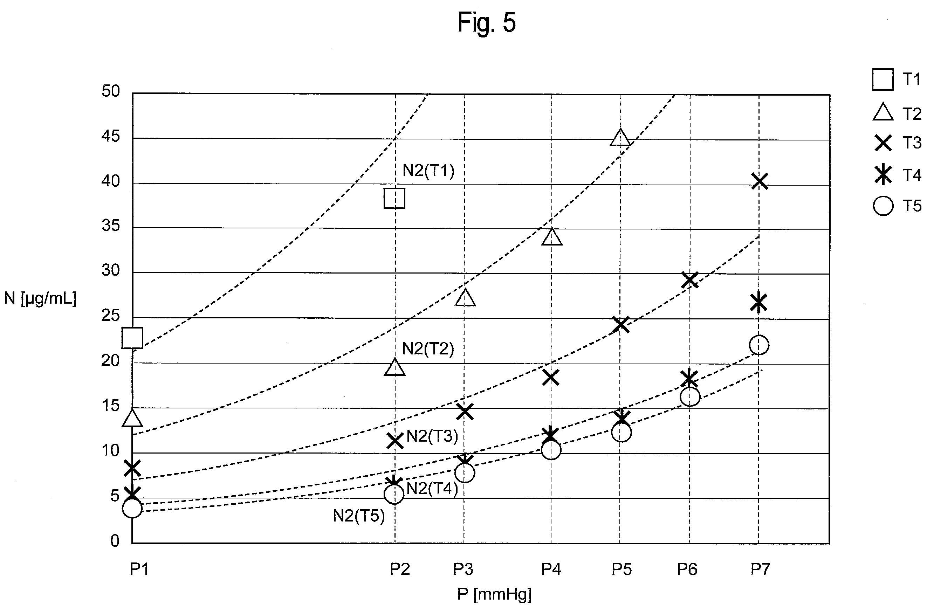

The function acquisition means 90 acquires respective functions $Fn(T1)$, $Fn(T2)$, $Fn(T3)$, $Fn(T4)$, and $Fn(T5)$ at a plurality of predetermined times after the initiation of the blood purification shown in Fig. 2, e.g., at times T1 to T5 (when a time at which the blood purification is initiated is assumed to be zero, T1 <T2<T3<T4<T5 is satisfied). For example, as shown in Fig. 4, the function acquisition means 90 has an acquisition means 100 and a calculation means 101. For example, as shown in Fig. 5, the acquisition means 100 sets the transmembrane pressure difference to a given value Pm, measures a solute leakage concentration Nm(Tn) at each of the times Tn = T1 to T5 after the initiation of the blood purification, repeats the above procedure a plurality of times, while varying a value of the given value Pm, and acquires the solute leakage concentrations N1(Tn) ... Nm(Tn) (where m is an integer of not less than 2) at each of the times Tn under the plurality of transmembrane pressure differences P1(Tn) ... Pm(Tn). The calculation means 101 obtains the function Fn from respective values of the solute leakage concentrations N1 (Tn) ... Nm(Tn) at each of the times Tn under the plurality of transmembrane pressure differences P1(Tn) ... Pm(Tn) acquired by the acquisition means 100.

Specifically, in Fig. 5, m = 7 is satisfied, the solute leakage concentrations N1(Tn), N2(Tn) ... N7(Tn) at each of the times Tn = T1, T2, T3, T4, and T5 are measured under P1 to P7, and the function Fn of the solute leakage concentration N(Tn) with respect to the transmembrane pressure difference P(Tn) at each of the times Tn is obtained from the respective measured values thereof (plots in the graph of Fig. 5). At this time, from the measured values (plots) in the graph of Fig. 5, approximate curves at the individual times Tn are obtained using exponential approximation, linear approximation, logarithmic approximation, or the like and determined to be the functions Fn(T1) to Fn(T5).

The function storage means 91 stores the functions Fn(T1) to Fn(T5) acquired by the function acquisition means 90.

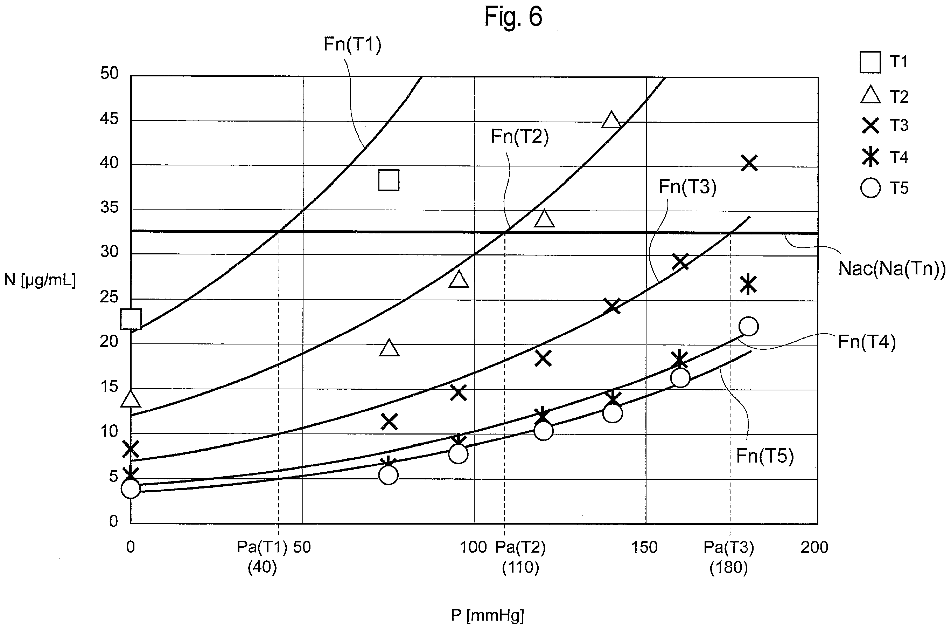

As shown in Fig. 6, the transmembrane pressure difference calculation means 92 obtains, on the basis of the functions Fn(T1) to Fn(T5) stored in the function storage means 91, the transmembrane pressure difference Pa(Tn) corresponding to the target solute leakage concentration Na(Tn) at each of the times Tn. For example, when the target solute leakage concentration Na(Tn) at each of the times T1 to T5 is assumed to have a given value Nac (e.g., 33 μg/mL in Fig. 6), the set transmembrane pressure difference Pa(Tn) (in Fig. 6, e.g., Pa(T1) = 40 mmHg, Pa(T2) = 110 mmHg, and Pa(T3 = 180 mmHg) corresponding thereto is obtained from the function Fn(Tn) at each of the times T1 to T5 to be determined. For example, the target solute leakage concentration Nac is obtained such that an amount of solute leakage during a total blood purification period corresponds to the target solute leakage amount. For example, the solute leakage concentration Nac is determined such that, when the target solute leakage amount during a blood purification period of 4 hours is 4 g, the amount of solute leakage per hour is 1 g. The amount of solute leakage at this time is obtained from a solute leakage concentration per unit time and from an amount of the dialysate discharged through the dialysate discharge circuit 51 per unit time. Note that, in the present embodiment, the transmembrane pressure difference calculation means 92 corresponds to a device which obtains a transmembrane pressure difference across a blood purification membrane and to a device which determines the transmembrane pressure difference across the blood purification membrane. A method for obtaining the transmembrane pressure difference across the blood purification membrane and a method for determining the transmembrane pressure difference across the blood purification membrane are each implemented through execution of a program stored in the storage portion of the control means 15.

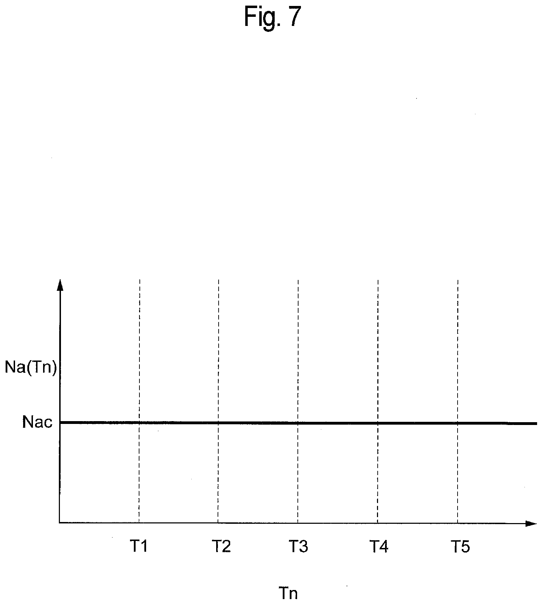

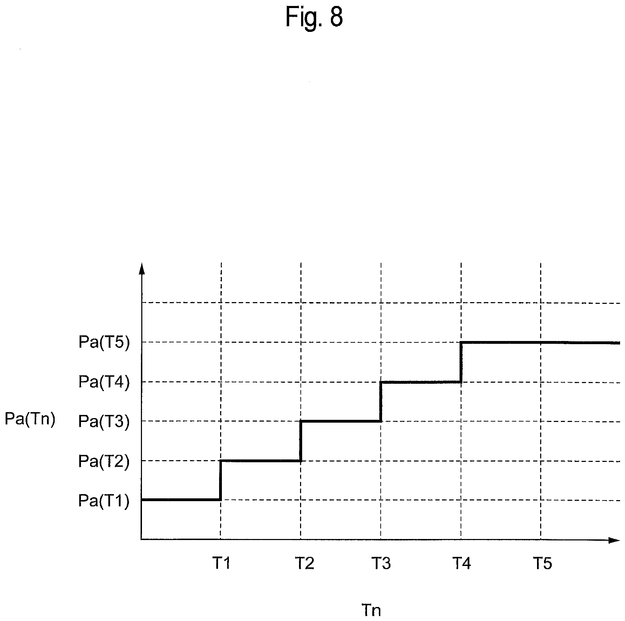

The transmembrane pressure difference control means 93 controls the transmembrane pressure difference at each of the times Tn during the blood purification such that the transmembrane pressure difference Pa(Tn) obtained by the transmembrane pressure difference calculation means 92 is achieved. For example, as shown in Figs. 7 and 8, the set transmembrane pressure differences Pa(T1), Pa(T2), Pa(T3), Pa(T4), and Pa(T5) are increased stepwise such that the solute leakage concentration at each of the times T1, T2, T3, T4, and T5 is equal to the given target solute leakage concentration Nac.

The transmembrane pressure difference control means 93 adjusts the transmembrane pressure difference pressure difference by adjusting the supply flow rate of the replacement fluid achieved by the replacement fluid pump 61 on the basis of detected values from at least the pressure sensors 44 and 55 in Fig. 1. Note that, by adjusting the supply flow rate of the replacement fluid achieved by the replacement fluid pump 61 on the basis of detected values from the pressure sensors 42 and 53 in addition to those from the pressure sensors 44 and 55, the transmembrane pressure difference can be adjusted. Note that the adjustment of the transmembrane pressure difference is not limited to that based on the adjustment of the supply flow rate of the replacement fluid. The transmembrane pressure difference may also be adjusted through adjustment of the blood-side flow rate or the dialysate-side flow rate.

Next, a description will be given of an example of the blood purification treatment using the blood purifying device 1.

First, the transmembrane pressure difference across the blood purification membrane 21 to be controlled during the blood purification is determined. First, the function acquisition means 90 of the control means 15 acquires the function Fn. At this time, for example, as shown in Fig. 5, the acquisition means 100 sets the given value Pm of the transmembrane pressure difference, measures the solute leakage concentration Nm(Tn) at each of the times Tn = T1 to T5 after the initiation of the blood purification, performs the measurement a plurality of times, while varying the value of the given value Pm, and acquires the solute leakage concentrations N1 (Tn) ... Nm(Tn) at each of the times Tn under the plurality of individual transmembrane pressure differences P1 (Tn) ... Pm(Tn) (where m is an integer of not less than 2). Then, from the respective values of the solute leakage concentrations N1(Tn) ... Nm(Tn) at each of the times Tn under the plurality of transmembrane pressure differences P1(Tn) ... Pm(Tn), the functions Fn(T1) to Fn(T5) at the individual times Tn = T1 to T5 shown in Fig. 2 are obtained. A step of acquiring the function Fn may be performed either before the initiation of the blood purification or during the blood purification. It may also be possible that data acquired during a given blood purification session is used when the function Fn is acquired during another blood purification session.

Then, the transmembrane pressure difference calculation means 92 of the control means 15 obtains, on the basis of the function Fn(Tn) shown in Fig. 6, the transmembrane pressure difference Pa(Tn) corresponding to the target solute leakage concentration Na(Tn) (given value Nac) at each of the times Tn, and sets the transmembrane pressure difference Pa(Tn) as the transmembrane pressure difference at each of the times Tn to be controlled during the blood purification.

Then, as shown in Fig. 1, respective puncture needles of the blood collection portion 30 and the blood return portion 32 are inserted into a patient, and the blood purification is initiated. The blood pump 40 of the blood circuit 11 is activated to pump out blood from the patient to the primary side of the blood purification membrane 21 of the blood purifier 10 through the blood collection circuit 31, and the blood flowing through the primary side of the blood purification membrane 21 is returned to the patient through the blood return circuit 33.

In the dialysate circuit 12, the dialysate supply pump 52, the dialysate discharge pump 54, or the like supplies the dialysate to the secondary side of the blood purification membrane 21 of the blood purifier 10 through the dialysate supply circuit 50, and the dialysate flowing through the secondary side of the blood purification membrane 21 is discharged through the dialysate discharge circuit 51. In the blood purifier 10, an unneeded component of the blood passes through the blood purification membrane 21 to be discharged by the dialysate. At this time, a portion of albumin as a specified useful solute also passes through the blood purification membrane 21 to be discharged.

To the blood flowing in the blood collection circuit 31 of the blood circuit 11, the anticoagulant is supplied from the anticoagulant supply circuit 14. Also, to the blood in the blood collection circuit 31, from the dialysate supply circuit 50, the replacement fluid (dialysate) is supplied at the predetermined flow rate by the replacement fluid circuit 13. The supply flow rate of the replacement fluid at this time is controlled by adjusting a flow rate (speed) of the replacement fluid pump 61.

During the blood purification, the transmembrane pressure difference control means 93 controls the transmembrane pressure difference at each of the times Tn during the blood purification. The transmembrane pressure difference at each of the times Tn is controlled to be equal to the set transmembrane pressure difference Pa(Tn) corresponding to the target solute leakage concentration Na(Tn) at each of the times Tn obtained by the transmembrane pressure difference calculation means 92. For example, as shown in Fig. 8, at each of the times T1 to T5 after the initiation of the blood purification, the set transmembrane pressure difference Pa(Tn) is increased stepwise. During the blood purification, the pressure sensor 42 or the pressure sensor 44 detects a pressure on the primary side of the blood purification membrane 21, while the pressure sensor 53 or the pressure sensor 55 detects a pressure on the secondary side of the blood purification membrane 21. The supply flow rate of the replacement fluid pump 61 of the replacement fluid circuit 13 is adjusted on the basis of at least the pressures across the blood purification membrane 21 detected by the pressure sensors 44 and 55 to control the transmembrane pressure difference across the blood purification membrane 21. Note that the supply flow rate of the replacement fluid pump 61 of the replacement fluid circuit 13 is adjusted on the basis of the pressures across the blood purification membrane 21 detected by the pressure sensors 42 and 53 in addition to the pressures across the blood purification membrane 21 detected by the pressure sensors 44 and 55 to control the transmembrane pressure difference across the blood purification membrane 21. At this time, the transmembrane pressure difference is controlled by, e.g., proportional control (P control) as feedback control.

According to the present embodiment, the control means 15 obtains, on the basis of the function Fn showing the relationship between the transmembrane pressure difference P(Tn) and the albumin leakage concentration N(Tn) at each of the plurality of times Tn after the blood purification, the transmembrane pressure difference Pa(Tn) corresponding to the target solute leakage concentration Na(Tn) at each of the times Tn, and controls the transmembrane pressure difference at each of the times Tn during the blood purification on the basis of the transmembrane pressure difference Pa(Tn). This entails no rapid change in transmembrane pressure difference and can inhibit leakage of a large amount of albumin from the blood through the blood purification membrane 21. In addition, since the transmembrane pressure difference at each of the times Tn during the blood purification is controlled on the basis of the transmembrane pressure difference Pa(Tn) corresponding to the target solute leakage concentration Na(Tn) at each of the times Tn, the amount of albumin leakage can be controlled. Moreover, by performing the control described above, not only albumin leakage can be sequentially adjusted, but also an amount of albumin leakage per a blood purification (therapy) session can be adjusted. Note that, in the present embodiment, the transmembrane pressure difference is controlled on the basis of the functions Fn at the plurality of five predetermined times Tn. However, it may also be possible to control the transmembrane pressure difference on the basis of the functions Fn at the plurality of four or less or six or more predetermined times Tn, or the function Fn at the one predetermined time Tn.

Since the blood purifying device 1 has the function acquisition means 90, the blood purifying device 1 can acquire the function Fn and control the transmembrane pressure difference based on the function Fn.

The function acquisition means 90 has the acquisition means 100 which acquires the solute leakage concentrations N1(Tn) ... Nm(Tn) at each of the times Tn under the individual transmembrane pressure differences P1(Tn) ... Pm(Tn) as the plurality of transmembrane pressure differences Pm (m is an integer of not less than 2) and the calculation means 101 which obtains the function Fn from the solute leakage concentrations N1(Tn) ... Nm(Tn) at each of the times Tn under the individual transmembrane pressure differences P1(Tn) ... Pm(Tn). This allows the function Fn to be appropriately be obtained.

The control means 15 controls the transmembrane pressure difference by varying the supply flow rate of the replacement fluid achieved by the replacement fluid pump 61. Accordingly, the transmembrane pressure difference can easily and precisely be controlled.

The control means 15 controls the transmembrane pressure difference by stepwise changing the transmembrane pressure difference. This allows the transmembrane pressure difference to be controlled through an easy control operation.

The control means 15 controls the transmembrane pressure difference by the proportional control (P control) as the feedback control. This allows the transmembrane pressure difference to be precisely controlled. Note that the control of the transmembrane pressure difference is not limited to that based on the proportional control. The transmembrane pressure difference may also be controlled by PI control or PID control.

The control means 15 controls the transmembrane pressure difference during the blood purification on the basis of the transmembrane pressure difference Pa(Tn) at each of the times Tn such that the solute leakage concentration N(Tn) during the blood purification is maintained at the given value Nac. This allows the total amount of albumin leakage per blood purification session to be easily and precisely controlled.

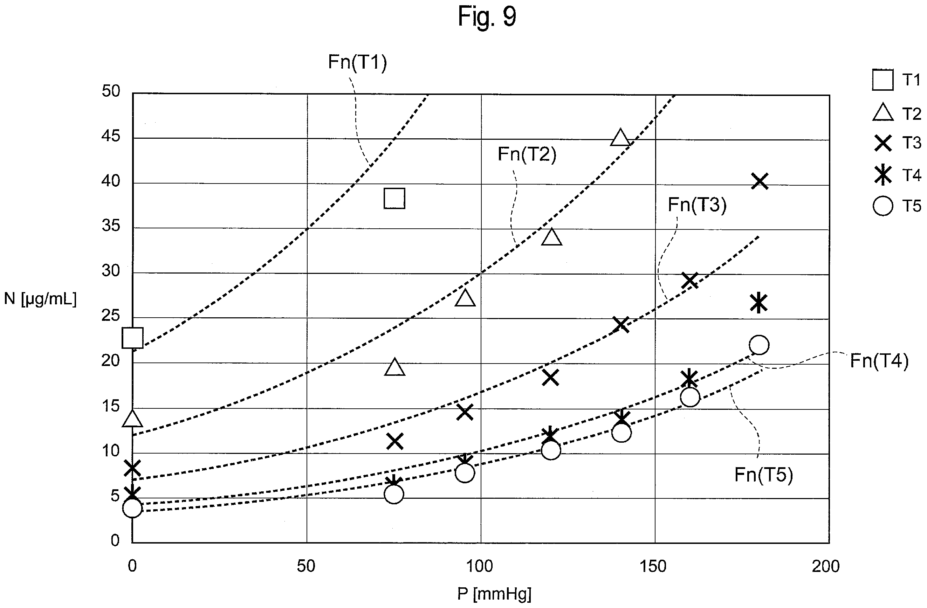

The function acquisition means 90 in the embodiment described above may also have a means that acquires the solute leakage concentration N(Tn) when the transmembrane pressure difference P(Tn) is changed at each of the times Tn after the initiation of the blood purification and obtains the function Fn. In such a case, as shown in, e.g., Fig. 9, it may also be possible to vary the transmembrane pressure difference P at each of the times T1 to T5, measure the solute leakage concentrations N at a plurality of points for each of the times T1 to T5, obtain approximate curves from the measured values, and determine the approximate curves as the functions Fn(T1) to Fn(T5). In such a case, the function Fn can easily be acquired. The function acquisition means 90 measures the solute leakage concentrations N1(Tn)... Nm(Tn) at each of the times Tn in the blood purifying device 1 and acquires required information. However, the function acquisition means 90 may also acquire the required information from the outside of the blood purifying device 1. At this time, it may also be possible to, e.g., cause a device other than the blood purifying device used for the therapy to acquire the function Fn, cause the blood purifying device used for the therapy to retrieve a program including the function Fn, cause the blood purifying device that has retrieved the program to execute the program, and thus control the transmembrane pressure difference and the solute leakage.

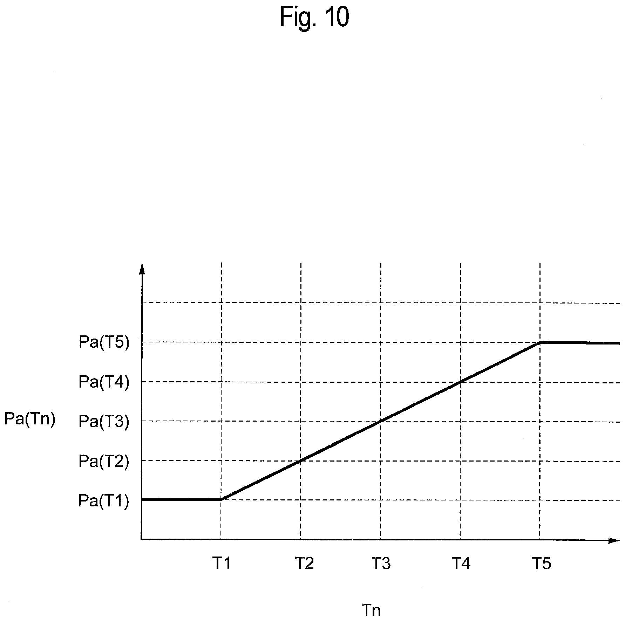

For example, as shown in Fig. 10, the control means 15 may also control the set transmembrane pressure difference Pa(Tn) by continuously varying the set transmembrane pressure difference Pa(Tn). In this case, the amount of albumin leakage per blood purification treatment (therapy) session can more accurately be adjusted.

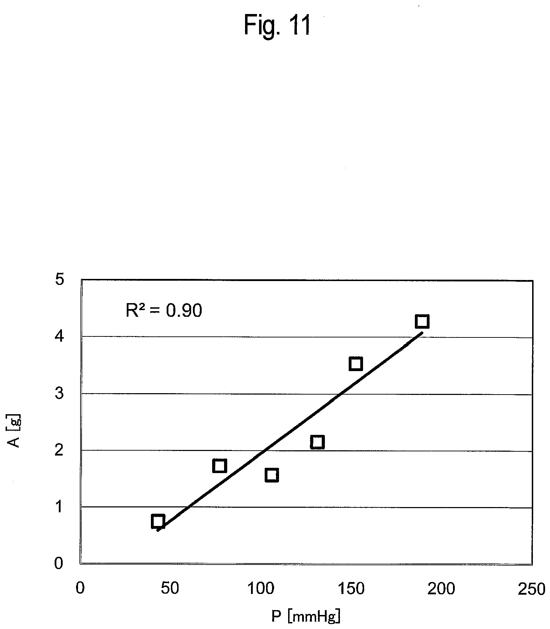

In the embodiment described above, as the blood purifier 10 of the blood purifying device 1, a blood purifier having a closer correlation between the average value of the transmembrane pressure differences and the amount of solute leakage may also be used. At this time, a filtration method for blood purification may be either constant-rate filtration in which the supply flow rate of the replacement fluid is held constant or constant-pressure filtration in which the transmembrane pressure difference is held constant. For example, as the blood purifier 10, a blood purifier in which a square value of a correlation coefficient between an average value Pi (where i is an integer of not less than 3) of the transmembrane pressure differences in the function Fn and a solute leakage amount Ai during 240 minutes after the initiation of the blood purification is not less than 0.3, preferably not less than 0.6, or more preferably not less than 0.9 may also be used. At this time, as shown in Fig. 11, it may also be possible to, e.g., plot a relationship between the average value Pi of the transmembrane pressure differences and the solute leakage amount Ai during 240 minutes after the initiation of the blood purification in a graph, obtain an approximate curve (linear approximation) using all the plots, and calculate the square value of the correlation coefficient from the approximate curve. Note that, in Fig. 11, measurement was performed under a constant-pressure filtration condition. By thus using the blood purifier 10 having the close correlation between the average value of the transmembrane pressure differences and the solute leakage amount, the transmembrane pressure difference can more precisely be controlled using the function Fn. In the correlation coefficient between the average value Pi (i is an integer of not less than 3) of the transmembrane pressure differences and the solute leakage amount Ai, i is set herein to an integer of not less than 3 because, when there are only two plots in the calculation of the correlation coefficient, the correlation coefficient inevitably becomes 1, and therefore the correlation described above is not established.

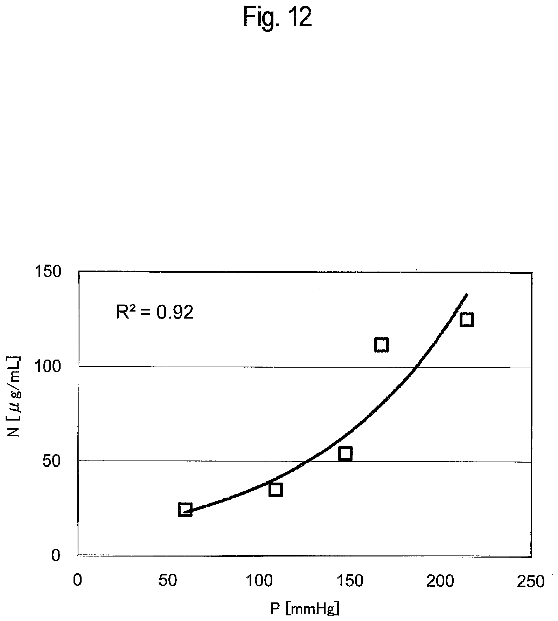

As the blood purifier 10, a blood purifier in which a square value of a correlation coefficient between a transmembrane pressure difference Pj (where j is an integer of not less than 3) and a solute leakage concentration Nj (where j is an integer of not less than 3) in the function Fn at S = 240 minutes as a time 240 minutes after the initiation of the blood purification is not less than 0.4 may also be used. At this time, a filtration method for blood purification may be either the constant-rate filtration or the constant-pressure filtration. At S = 120 minutes as a time 120 minutes after the initiation of the blood purification, the blood purifier 10 in which the square value of the correlation coefficient between the transmembrane pressure difference Pj (where j is an integer of not less than 3) and the solute leakage concentration Nj (where j is an integer of not less than 3) is not less than 0.6 may also be used. At S = 60 minutes as a time 60 minutes after the initiation of the blood purification, the blood purifier 10 in which the square value of the correlation coefficient between the transmembrane pressure difference Pj (where j is an integer of not less than 3) and the solute leakage concentration Nj (where j is an integer of not less than 3) is not less than 0.8 may also be used. At S = 30 minutes as a time 30 minutes after the initiation of the blood purification, the blood purifier 10 in which the square value of the correlation coefficient between the transmembrane pressure difference Pj (where j is an integer of not less than 3) and the solute leakage concentration Nj (where j is an integer of not less than 3) is not less than 0.9 may also be used. At this time, it may also be possible to, e.g., plot a relationship between the transmembrane pressure difference Pj and the solute leakage concentration Nj in the graph as shown in Fig. 12, obtain an approximate curve (exponential approximation) using all the plots, and calculate the square value of the correlation coefficient from the approximate curve. Note that, in Fig. 12, measurement was performed under a constant-pressure filtration condition. By thus using the blood purifier 10 having the close correlation between the transmembrane pressure difference and the solute leakage concentration, the transmembrane pressure difference can more precisely be controlled using the function Fn. In the correlation coefficient between the transmembrane pressure difference Pj (j is an integer of not less than 3) and the solute leakage concentration Nj, j is set herein to an integer of not less than 3 because, when there are only two plots in the calculation of the correlation coefficient, the correlation coefficient inevitably becomes 1, and therefore the correlation described above is not established.

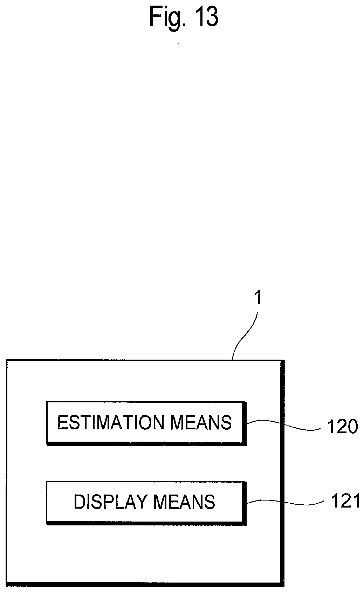

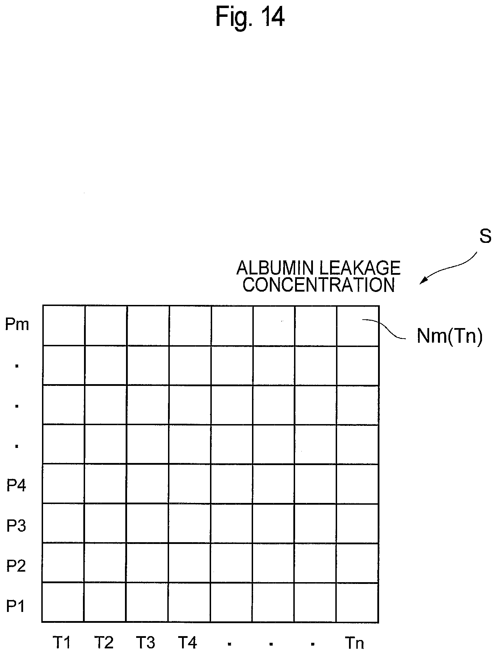

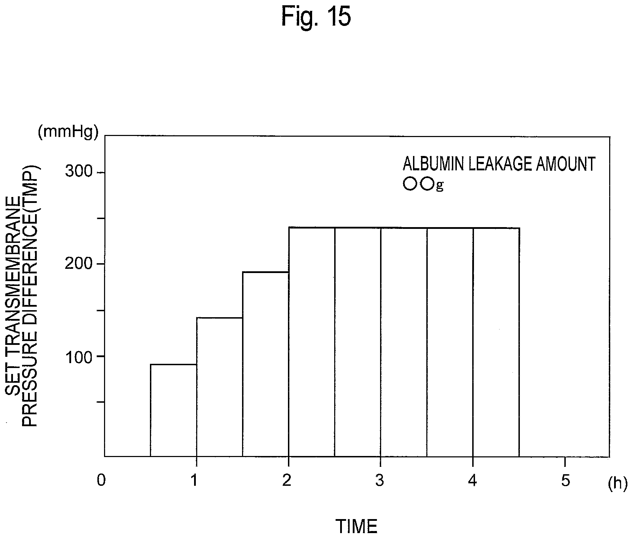

In the embodiment described above, as shown in Fig. 13, the blood purifying device 1 may also include an estimation means 120 which estimates a total amount of albumin leakage throughout the blood purification and a display means 121 which displays the amount of albumin leakage. For example, the estimation means 120 produces, from the solute leakage concentrations N1(Tn) ... Nm(Tn) under the plurality of transmembrane pressure differences P1(Tn) ... Pm(Tn) (where m is an integer of not less than 2) at each of the times Tn after the initiation of the blood purification, a data map S showing solute leakage concentrations N1(Tn) ... Nm(Tn) in a combination of each of the times Tn and the transmembrane pressure differences P1(Tn) ... Pm(Tn) as shown in Fig. 14, and estimates the total amount of albumin leakage during the blood purification from the data map S and from the set transmembrane pressure difference set for each of the times. The display means 121 displays, as shown in, e.g., Fig. 15, the estimated amount of albumin leakage, the set transmembrane pressure difference at each of the times during the blood purification, and the like.

The estimation means 120 may also estimate the solute leakage amount throughout the entire blood purification or per unit time from the function Fn and from the target transmembrane pressure difference set for each of the predetermined times without using the data map S. In such a case, it may also be possible to obtain the solute leakage concentration at each of the predetermined times using the function Fn from the target transmembrane pressure difference set for each of the predetermined times and estimate the solute leakage amount throughout the entire blood purification or per unit time from the solute leakage concentration.

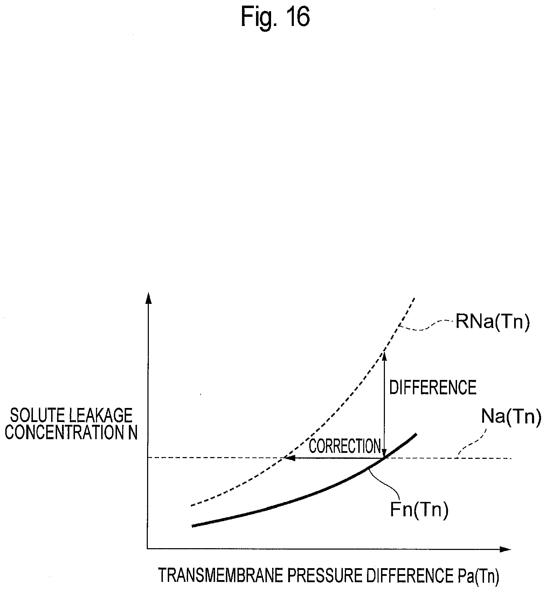

In the embodiment described above, it may also be possible to correct the transmembrane pressure difference Pa(Tn) calculated on the basis of the function Fn. As shown in Fig. 16, when the solute leakage concentration is actually measured, there may be a case where there is a difference between the actually measured solute leakage concentration and the target solute leakage concentration. Such a situation may be encountered when, e.g., the accuracy of the function Fn is insufficient. Accordingly, it may also be possible to, e.g., calculate, from an actually measured value RNa(Tn) of the solute leakage concentration under the transmembrane pressure difference Pa(Tn) at each of the one or more predetermined times Tn after the initiation of the blood purification, a ratio W between the target solute leakage concentration Na(Tn) and the actually measured value RNa(Tn) for each of the times Tn, multiply the transmembrane pressure difference Pa(Tn) by the ratio W to obtain a transmembrane pressure difference Pa'(Tn), and control the transmembrane pressure difference during the blood purification on the basis of the transmembrane pressure difference Pa'(Tn). In such a case, the actually measured value RNa(Tn) of the solute leakage concentration corresponding to the transmembrane pressure difference Pa(Tn) at each of the predetermined times Tn is measured using a concentration sensor, and the actually measured values RNa(T1) ... RNa(Tn) measured by the concentration sensor are output to the control means 15. Note that the concentration sensor may also be provided in, e.g., the dialysate discharge circuit 51. The control means 15 calculates the ratio W (W = Na(Tn)/RNa(Tn)) between the target solute leakage concentration Na(Tn) and the actually measured value RNa(Tn) for each of the predetermined times Tn, and multiplies the transmembrane pressure difference Pa(Tn) by the ratio W to obtain the transmembrane pressure difference Pa'(Tn). The control means 15 determines the transmembrane pressure difference Pa'(Tn) to be the new set transmembrane pressure difference Pa'(Tn) resulting from the correction and controls the transmembrane pressure difference at each of the predetermined times Tn.

According to the example described above, the transmembrane pressure difference Pa(Tn) is corrected on the basis of the actually measured value of the solute leakage concentration, and the transmembrane pressure difference at each of the predetermined times Tn is controlled on the basis of the new transmembrane pressure difference Pa'(Tn). This allows the transmembrane pressure difference throughout the entire blood purification treatment to be more precisely controlled. As a result, it is possible to precisely control the solute leakage amount from the blood through the blood purification membrane 21 and control the blood of the patient to, e.g., a complicating disease preventive region which is determined by an amount of a disease agent removed from the blood.

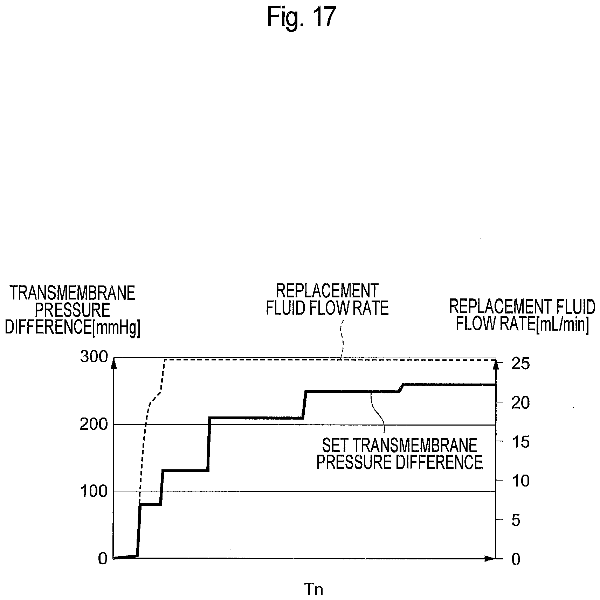

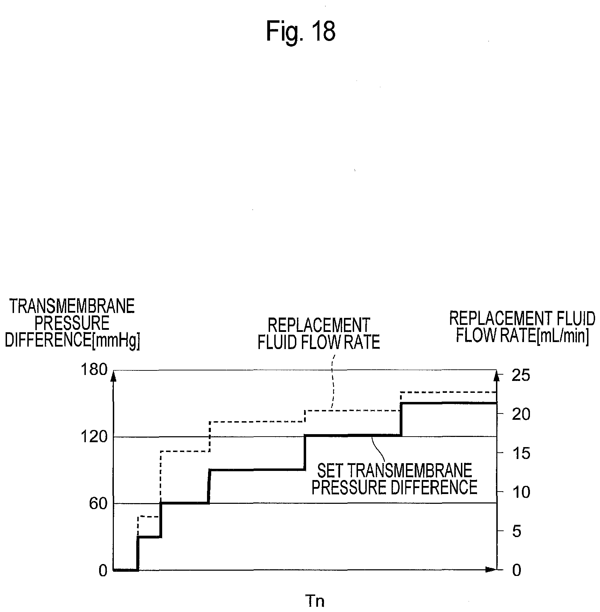

In the embodiment described above, the transmembrane pressure difference is controlled using the supply flow rate of the replacement fluid. However, the supply flow rate of the replacement fluid has an upper limit (arbitrarily set to be not more than the dialysate-side flow rate). Accordingly, when, e.g., the set transmembrane pressure difference to be controlled rapidly increases and the supply flow rate of the replacement fluid corresponding to the set transmembrane pressure difference reaches the upper limit as shown in Fig. 17, the supply flow rate of the replacement fluid may no longer increase beyond the upper limit and the set transmembrane pressure difference may not be able to be precisely achieved. In this case, it may also be possible to change the transmembrane pressure difference Pa(Tn), while maintaining the average value P of the transmembrane pressure differences throughout the entire blood purification treatment unchanged and preventing the supply flow rate of the replacement fluid at each of the predetermined times Tn from reaching the upper limit, to obtain a transmembrane pressure difference Pa"(Tn), and control the transmembrane pressure difference during the blood purification on the basis of the transmembrane pressure difference Pa"(Tn). In such a case, for example, the average value P of the transmembrane pressure differences throughout the entire blood purification treatment is obtained from the transmembrane pressure difference Pa(Tn) at each of the predetermined times Tn, and the transmembrane pressure difference throughout the entire blood purification treatment or at each of the predetermined times Tn is adjusted so as to prevent the supply flow rate of the replacement fluid from reaching the upper limit, as shown in Fig. 18. In such a case, when, e.g., the supply flow rate of the replacement fluid at each of the predetermined times Tn exceeds an arbitrarily set threshold immediately after the control, it is determined that there is a high possibility that the supply flow rate of the replacement fluid reaches the upper limit during the blood purification treatment. Then, the transmembrane pressure difference throughout the entire blood purification treatment or at each of the predetermined time Tn is adjusted so as to prevent the supply flow rate of the replacement fluid from reaching the upper limit and maintain the average value P of the transmembrane pressure differences throughout the entire blood purification treatment obtained from the transmembrane pressure difference Pa(Tn) at each of the predetermined times Tn. In another example, when the supply flow rate of the replacement fluid at each of the predetermined times Tn reaches the upper limit after the control, an amount of the replacement fluid to be supplied is temporarily returned to a value before the control, and then the transmembrane pressure difference throughout the entire blood purification treatment or at each of the predetermined times Tn is adjusted so as to prevent the supply flow rate of the replacement fluid from reaching the upper limit and maintain the average value P of the transmembrane pressure differences throughout the entire blood purification treatment obtained from the transmembrane pressure difference Pa(Tn) at each of the predetermined times. At this time, the average value of the transmembrane pressure differences throughout the entire blood purification treatment after the adjustment remains unchanged and is maintained at the average value P. By doing so, it is possible to adjust the transmembrane pressure difference exactly as set and consequently precisely control the solute leakage amount.

In the embodiment described above, a circuit configuration of the blood purifying device 1 is not limited to the configuration described above in the embodiment. For example, while the replacement fluid circuit 13 supplies the replacement fluid to the blood collection circuit 31 above, the replacement fluid circuit 13 may also supply the replacement fluid to the blood return circuit 33 or may also supply the replacement fluid to each of the blood collection circuit 31 and the blood return circuit 33. Also, while the amount of albumin leakage through the blood purification membrane 21 is controlled above, it may also be possible to control another solute in the blood, e.g., an amount of leakage of a protein having a molecular weight of 10,000 Da to 100,000 Da. The blood purifier 10 may also have the blood purification membrane 21 other than the hollow fiber membrane. The present invention is not limited to the blood purifying device for performing hemodiafiltration (HDF), and is also applicable to a blood purifying device for performing hemodialysis (HD) or hemofiltration (HF) and further to a blood purifying device for performing continuous hemofiltration, continuous hemodiafiltration, SCUF (slow continuous ultrafiltration), or the like.

Industrial Applicability

The present invention is useful in controlling an amount of leakage of a specified solute in blood when the blood is purified by a blood purification membrane, while inhibiting the leakage of the solute.

Reference Signs List

| 1 | Blood purifying device |
|---|---|
| 10 | Blood purifier |
| 11 | Blood circuit |
| 13 | Replacement fluid circuit |
| 15 | Control means |
| 21 | Blood purification membrane |

**Claims**

1. A blood purifying device (1) for purifying blood using a blood purifier (10), the blood purifying device (1) comprising:

    control means (15) configured to control a transmembrane pressure difference across a blood purification membrane (21) of the blood purifier (10),
    function acquisition means (90) configured to acquire a function Fn,
    wherein the function acquisition means (90) includes:

        acquisition means (100) configured to acquire respective solute leakage concentrations N1(Tn) ... Nm(Tn) at each of the one or more predetermined times Tn for respective transmembrane pressure differences P1(Tn) ... Pm(Tn) as a plurality of transmembrane pressure differences Pm, wherein m is an integer of not less than 2; and
        calculation means (92) configured to obtain the function Fn from respective values of the solute leakage concentrations N1(Tn) ... Nm(Tn) at each of the one or more predetermined times Tn for the respective transmembrane pressure differences P1(Tn) ... Pm(Tn) acquired by the acquisition means (100),

    wherein on the basis of the function Fn given by the numerical expression:

    $$N(Tn) = Fn(P(Tn), Tn)$$

    wherein n is an integer of not less than 1, and
    where the numerical expression shows a relationship between the transmembrane pressure difference P(Tn) and the solute leakage concentration N(Tn) of a predetermined solute in the blood at each of one or more predetermined times Tn after initiation of blood purification, the control means obtains a transmembrane pressure difference Pa(Tn) corresponding to a target solute leakage concentration Na(Tn) at each of the one or more predetermined times Tn, and controls the transmembrane pressure difference during the blood purification on the basis of the transmembrane pressure difference Pa(Tn).

2. The blood purifying device (1) according to claim 1, wherein the function acquisition means (90) includes means configured to acquire the solute leakage concentration N(Tn) when the transmembrane pressure difference P(Tn) is changed for each of the one or more predetermined times Tn after the initiation of the blood purification to obtain the function Fn.

3. The blood purifying device (1) according to any one of claims 1 or 2, wherein the blood purifier (10) is configured such that the square value of the correlation coefficient between the average value Pi, wherein i is an integer of not less than 3, of the transmembrane pressure differences and the solute leakage amount Ai during 240 minutes after the initiation of the blood purification is not less than 0.3.

4. The blood purifying device (1) according to any one of claims 1 to 3, wherein the blood purifier (10) is configured such that the square value of the correlation coefficient between the transmembrane pressure difference Pj, wherein j is an integer of not less than 3, and the solute leakage concentration Nj at S = 240 minutes as a time 240 minutes after the initiation of the blood purification is not less than 0.4.

5. The blood purifying device (1) according to any one of claims 1 to 4, further comprising:

a blood circuit (11) configured to supply/discharge the blood to/from the blood purifier (10);

a dialysate circuit (12) configured to supply/discharge the dialysate to/from the blood purifier (10); and

a replacement fluid circuit (13) configured to supply a replacement fluid to a side of the blood circuit upstream of the blood purifier (10) and/or to a side of the blood circuit downstream of the blood purifier (10), wherein the control means (15) is configured to control the transmembrane pressure difference by changing a supply flow rate of the replacement fluid.

6. The blood purifying device (1) according to any one of claims 1 to 5, wherein the control means (15) is configured to control the transmembrane pressure difference by stepwise or continuously changing the transmembrane pressure difference.

7. The blood purifying device (1) according to any one of claims 1 to 6, wherein the control means (15) is configured to control the transmembrane pressure difference by feedback control.

8. The blood purifying device (1) according to any one of claims 1 to 7, wherein the control means (15) is configured to control the transmembrane pressure difference during the blood purification on the basis of the transmembrane pressure difference $Pa(Tn)$ at each of the times $Tn$ so as to maintain the solute leakage concentration $N(Tn)$ during the blood purification constant.

9. The blood purifying device (1) according to any one of claims 1 to 8, further comprising:
estimation means (120) configured to produce, from data of the solute leakage concentrations $N1(Tn) ... Nm(Tn)$ under the plurality of transmembrane pressure differences $P1(Tn) ... Pm(Tn)$ (where m is an integer of not less than 2) at each of the one or more predetermined times $Tn$ after the initiation of the blood purification, a data map showing the solute leakage concentrations $N1(Tn) ... Nm(Tn)$ in a combination of each of the predetermined times $Tn$ and the transmembrane pressure differences $P1(Tn) ... Pm(Tn)$, and to estimate the solute leakage amount throughout the entire blood purification or per unit time from the data map and from the transmembrane pressure difference set for each of the times.

10. The blood purifying device (1) according to any one of claims 1 to 8, further comprising:
an estimation means (120) configured to estimate the solute leakage amount throughout the entire blood purification or per unit time from the function $Fn$ and from a target transmembrane pressure difference set for each of the predetermined times.

11. The blood purifying device (1) according to any one of claims 1 to 10, wherein the control means (15) is configured to calculate, from an actually measured value $RNa(Tn)$ of the solute leakage concentration under the transmembrane pressure difference $Pa(Tn)$ at each of the one or more predetermined times $Tn$ after the initiation of the blood purification, a ratio between the target solute leakage concentration $Na(Tn)$ and the actually measured value at each of the predetermined times $Tn$, to multiply the transmembrane pressure difference $Pa(Tn)$ by the ratio to obtain a transmembrane pressure difference $Pa'(Tn)$, and to control the transmembrane pressure difference during the blood purification on the basis of the transmembrane pressure difference $Pa'(Tn)$.

12. The blood purifying device (1) according to any one of claims 1 to 10, wherein the control means (15) is configured to change the transmembrane pressure difference $Pa(Tn)$, while maintaining the average value P of the transmembrane pressure differences throughout entire blood purification treatment unchanged and preventing the supply flow rate of the replacement fluid at each of the predetermined times $Tn$ from reaching an upper limit, to obtain a transmembrane pressure difference $Pa''(Tn)$, and to control the transmembrane pressure difference during the blood purification on the basis of the transmembrane pressure difference $Pa''(Tn)$.

13. A method for obtaining a transmembrane pressure difference across a blood purification membrane (21), when blood is purified using a blood purifier (10), which comprises the steps of:
obtaining, from a function $Fn$ given by a numerical expression:
$N(Tn) = Fn(P(Tn),Tn)$, wherein n is an integer of not less than 1 where the numerical expression shows a relationship between a transmembrane pressure difference $P(Tn)$ and a solute leakage concentration $N(Tn)$ of a predetermined solute in the blood at each of one or more predetermined times $Tn$ after initiation of blood purification, a transmembrane pressure difference $Pa(Tn)$ corresponding to a target solute leakage concentration $Na(Tn)$ at each of the one or more predetermined times $Tn$, wherein the function $Fn$ is acquired by a function acquisition means (90), wherein the function acquisition means (90) includes:

acquisition means (100) configured to acquire respective solute leakage concentrations N1(Tn) ... Nm(Tn) at each of the one or more predetermined times Tn for respective transmembrane pressure differences P1(Tn) ... Pm(Tn) as a plurality of transmembrane pressure differences Pm, wherein m is an integer of not less than 2; and calculation means (92) configured to obtain the function Fn from respective values of the solute leakage concentrations N1(Tn) ... Nm(Tn) at each of the one or more predetermined times Tn for the respective transmembrane pressure differences P1(Tn) ... Pm(Tn) acquired by the acquisition means (100),

under the proviso that methods for the treatment of the human and animal body by therapy and surgery are excluded.

14. The method for obtaining the transmembrane pressure difference across the blood purification membrane (21) according to claim 13, wherein the blood purifier (10) is configured such that the square value of the correlation coefficient between the average value Pi, wherein i is an integer of not less than 3, of the transmembrane pressure differences and the solute leakage amount Ai during 240 minutes after the initiation of the blood purification is not less than 0.3.

15. The method for obtaining the transmembrane pressure difference across the blood purification membrane (21) according to claim 13, wherein the blood purifier (10) is configured such that the square value of the correlation coefficient between the transmembrane pressure difference Pj, wherein j is an integer of not less than 3, and the solute leakage concentration Nj at S = 240 minutes as a time 240 minutes after the initiation of the blood purification is not less than 0.4.

16. The method for obtaining the transmembrane pressure difference across the blood purification membrane (21) according to any one of claims 13 to 15, wherein the method calculates, from an actually measured value RNa(Tn) of the solute leakage concentration under the transmembrane pressure difference Pa(Tn) at each of the one or more predetermined times Tn after the initiation of the blood purification, the ratio between the target solute leakage concentration Na(Tn) and the actually measured value at each of the predetermined times Tn, and multiplies the transmembrane pressure difference Pa(Tn) by the ratio to obtain a transmembrane pressure difference Pa'(Tn).

17. The method for obtaining the transmembrane pressure difference across the blood purification membrane (21) according to any one of claims 13 to 15, wherein the method changes the transmembrane pressure difference Pa(Tn), while maintaining the average value P of the transmembrane pressure differences throughout entire blood purification treatment unchanged and preventing the supply flow rate of the replacement fluid at each of the predetermined times Tn from reaching an upper limit, to obtain a transmembrane pressure difference Pa"(Tn).

18. The method for obtaining the transmembrane pressure difference across the blood purification membrane (21) according to claim 13, further comprising the step of determining a transmembrane pressure difference during the blood purification on the basis of the transmembrane pressure difference Pa(Tn).

19. The method for determining the transmembrane pressure difference across the blood purification membrane (21) according to claim 18, wherein the blood purifier (10) is configured such that the square value of the correlation coefficient between the average value Pi, wherein i is an integer of not less than 3, of the transmembrane pressure differences and the solute leakage amount Ai during 240 minutes after the initiation of the blood purification is not less than 0.3.

20. The method for determining the transmembrane pressure difference across the blood purification membrane (21) according to claim 18, wherein the blood purifier (10) is configured such that the square value of the correlation coefficient between the transmembrane pressure difference Pj, wherein j is an integer of not less than 3, and the solute leakage concentration Nj at S = 240 minutes as a time 240 minutes after the initiation of the blood purification is not less than 0.4.

21. The method for determining the transmembrane pressure difference across the blood purification membrane (21) according to any one of claims 18 to 20, wherein the method calculates, from an actually measured value RNa(Tn) of the solute leakage concentration under the transmembrane pressure difference Pa(Tn) at each of the one or more predetermined times Tn after the initiation of the blood purification, a ratio between the target solute leakage concentration Na(Tn) and the actually measured value at each of the predetermined times Tn, multiplies the transmembrane pressure difference Pa(Tn) by the ratio to obtain a transmembrane pressure difference Pa'(Tn), and determines the transmembrane pressure difference Pa'(Tn) to be the transmembrane pressure difference during the blood purification.

**22.** The method for determining the transmembrane pressure difference across the blood purification membrane (21) according to any one of claims 18 to 20, wherein the method changes the transmembrane pressure difference $Pa(Tn)$, while maintaining the average value $P$ of the transmembrane pressure differences throughout entire blood purification treatment unchanged and preventing the supply flow rate of the replacement fluid at each of the predetermined times $Tn$ from reaching an upper limit, to obtain a transmembrane pressure difference $Pa''(Tn)$, and determines the transmembrane pressure difference $Pa''(Tn)$ to be the transmembrane pressure difference during the blood purification.

**23.** A device for obtaining a transmembrane pressure difference across a blood purification membrane (21) when blood is purified using a blood purifier (10), wherein the device obtains, from a function $Fn$ given by a numerical expression: $N(Tn) = Fn(P(Tn),Tn)$, wherein n is an integer of not less than 1,

where the numerical expression shows a relationship between a transmembrane pressure difference $P(Tn)$ and a solute leakage concentration $N(Tn)$ of a predetermined solute in the blood at each of one or more predetermined times $Tn$ after initiation of blood purification, a transmembrane pressure difference $Pa(Tn)$ corresponding to a target solute leakage concentration $Na(Tn)$ at each of the one or more predetermined times $Tn$, wherein function $Fn$ is acquired by a function acquisition means (90),
wherein
the function acquisition means (90) includes:

acquisition means (100) configured to acquire respective solute leakage concentrations $N1(Tn)$ ... $Nm(Tn)$ at each of the one or more predetermined times $Tn$ for respective transmembrane pressure differences $P1(Tn)$ ... $Pm(Tn)$ as a plurality of transmembrane pressure differences $Pm$, wherein m is an integer of not less than 2; and
calculation means (92) configured to obtain the function $Fn$ from respective values of the solute leakage concentrations $N1(Tn)$ ... $Nm(Tn)$ at each of the one or more predetermined times $Tn$ for the respective transmembrane pressure differences $P1(Tn)$ ... $Pm(Tn)$ acquired by the acquisition means (100).

**24.** The device for obtaining the transmembrane pressure difference across the blood purification membrane (21) according to claim 23, wherein the blood purifier (10) is configured such that the square value of the correlation coefficient between the average value $Pi$, wherein i is an integer of not less than 3, of the transmembrane pressure differences and the solute leakage amount $Ai$ during 240 minutes after the initiation of the blood purification is not less than 0.3.

**25.** The device for obtaining the transmembrane pressure difference across the blood purification membrane (21) according to claim 23, wherein the blood purifier (10) is configured such that the square value of the correlation coefficient between the transmembrane pressure difference $Pj$, wherein j is an integer of not less than 3, and the solute leakage concentration $Nj$ at $S = 240$ minutes as a time 240 minutes after the initiation of the blood purification is not less than 0.4.

**26.** The device for obtaining the transmembrane pressure difference across the blood purification membrane (21) according to any one of claims 23 to 25, wherein the device is configured to calculate, from the actually measured value $RNa(Tn)$ of the solute leakage concentration under the transmembrane pressure difference $Pa(Tn)$ at each of the one or more predetermined times $Tn$ after the initiation of the blood purification, the ratio between the target solute leakage concentration $Na(Tn)$ and the actually measured value at each of the predetermined times $Tn$, and to multiply the transmembrane pressure difference $Pa(Tn)$ by the ratio to obtain a transmembrane pressure difference $Pa'(Tn)$.

**27.** The device for obtaining the transmembrane pressure difference across the blood purification membrane (21) according to any one of claims 23 to 26, wherein the device is configured to change the transmembrane pressure difference $Pa(Tn)$, while maintaining the average value $P$ of the transmembrane pressure differences throughout entire blood purification treatment unchanged and preventing the supply flow rate of the replacement fluid at each of the predetermined times $Tn$ from reaching an upper limit, to obtain a transmembrane pressure difference $Pa''(Tn)$.

**28.** A program for causing a computer (15) to implement a method for obtaining a transmembrane pressure difference across a blood purification membrane when blood is purified using a blood purifier (10), the method comprising a step of:
obtaining, from a function $Fn$ given by a numerical expression:

N(Tn) = Fn(P(Tn),Tn), wherein n is an integer of not less than 1,

where the numerical expression shows a relationship between a transmembrane pressure difference P(Tn) and a solute leakage concentration N(Tn) of a predetermined solute in the blood at each of one or more predetermined times Tn after initiation of blood purification, a transmembrane pressure difference Pa(Tn) corresponding to a target solute leakage concentration Na(Tn) at each of the one or more predetermined times Tn, wherein the function Fn is acquired by a function acquisition means (90), wherein the function acquisition means (90) includes:

acquisition means (100) configured to acquire respective solute leakage concentrations N1(Tn) ... Nm(Tn) at each of the one or more predetermined times Tn for respective transmembrane pressure differences P1(Tn) ... Pm(Tn) as a plurality of transmembrane pressure differences Pm (where m is an integer of not less than 2); and

calculation means (92) configured to obtain the function Fn from respective values of the solute leakage concentrations N1(Tn) ... Nm(Tn) at each of the one or more predetermined times Tn for the respective transmembrane pressure differences P1(Tn) ... Pm(Tn) acquired by the acquisition means (100).

29. The program according to claim 28, wherein, the blood purifier (10) is configured such that the square value of the correlation coefficient between the average value Pi, wherein i is an integer of not less than 3, of the transmembrane pressure differences and a solute leakage amount Ai during 240 minutes after the initiation of the blood purification is not less than 0.3.

30. The program according to claim 28, wherein, the blood purifier (10) configured such that the square value of the correlation coefficient between the transmembrane pressure difference Pj, wherein j is an integer of not less than 3, and a solute leakage concentration Nj at S = 240 minutes as a time 240 minutes after the initiation of the blood purification is not less than 0.4.

31. The program according to any one of claims 28 to 30, wherein the program calculates, from an actually measured value RNa(Tn) of the solute leakage concentration under the transmembrane pressure difference Pa(Tn) at each of the one or more predetermined times Tn after the initiation of the blood purification, a ratio between the target solute leakage concentration Na(Tn) and the actually measured value at each of the predetermined times Tn, and multiplies the transmembrane pressure difference Pa(Tn) by the ratio to obtain a transmembrane pressure difference Pa'(Tn).

32. The program according to any one of claims 28 to 30, wherein the program changes the transmembrane pressure difference Pa(Tn), while maintaining the average value P of the transmembrane pressure differences throughout entire blood purification treatment unchanged and preventing the supply flow rate of the replacement fluid at each of the predetermined times Tn from reaching an upper limit, to obtain a transmembrane pressure difference Pa"(Tn).

33. The program according to claim 28 further comprising the step of determining a transmembrane pressure difference during the blood purification on the basis of the transmembrane pressure difference Pa(Tn).

34. The program according to claim 33, wherein the blood purifier (10) is configured such that the square value of the correlation coefficient between the average value Pi, wherein i is an integer of not less than 3, of the transmembrane pressure differences and the solute leakage amount Ai during 240 minutes after the initiation of the blood purification is not less than 0.3.

35. The program according to claim 33, wherein the blood purifier (10) is configured such that the square value of the correlation coefficient between the transmembrane pressure difference Pj, wherein j is an integer of not less than 3, and a solute leakage concentration Nj at S = 240 minutes as a time 240 minutes after the initiation of the blood purification is not less than 0.4.

36. The program according to any one of claims 33 to 35, wherein the program calculates, from an actually measured value RNa(Tn) of the solute leakage concentration under the transmembrane pressure difference Pa(Tn) at each of the one or more predetermined times Tn after the initiation of the blood purification, a ratio between the target solute leakage concentration Na(Tn) and the actually measured value at each of the predetermined times Tn, multiplies the transmembrane pressure difference Pa(Tn) by the ratio to obtain a transmembrane pressure difference Pa'(Tn), and determines the transmembrane pressure difference Pa'(Tn) to be the transmembrane pressure difference during the blood purification.

37. The program according to any one of claims 33 to 35, wherein the program changes the transmembrane pressure difference Pa(Tn), while maintaining the average value P of the transmembrane pressure differences throughout entire blood purification treatment unchanged and preventing the supply flow rate of the replacement fluid at each of the predetermined times Tn from reaching an upper limit, to obtain a transmembrane pressure difference Pa"(Tn), and determines the transmembrane pressure difference Pa"(Tn) to be the transmembrane pressure difference during the blood purification.

38. A device for determining a transmembrane pressure difference across a blood purification membrane (21) when blood is purified using a blood purifier (10), wherein the device obtains, on the basis of a function Fn given by a numerical expression:
N(Tn) = Fn(P(Tn),Tn) wherein n is an integer of not less than 1,

   where the numerical expression shows a relationship between a transmembrane pressure difference P(Tn) and a solute leakage concentration N(Tn) of a predetermined solute in the blood at each of one or more predetermined times Tn after initiation of blood purification, a transmembrane pressure difference Pa(Tn) corresponding to a target solute leakage concentration Na(Tn) at each of the one or more predetermined times Tn, and determines a transmembrane pressure difference during the blood purification on the basis of the transmembrane pressure difference Pa(Tn),
   wherein the function Fn is acquired by a function acquisition means (90),
   wherein
   the function acquisition means (90) includes:

      acquisition means (100) configured to acquire respective solute leakage concentrations N1(Tn) ... Nm(Tn) at each of the one or more predetermined times Tn for respective transmembrane pressure differences P1(Tn) ... Pm(Tn) as a plurality of transmembrane pressure differences Pm (where m is an integer of not less than 2); and
      calculation means (92) configured to obtain the function Fn from respective values of the solute leakage concentrations N1(Tn) ... Nm(Tn) at each of the one or more predetermined times Tn for the respective transmembrane pressure differences P1(Tn) ... Pm(Tn) acquired by the acquisition means (100).

39. The device for determining the transmembrane pressure difference across the blood purification membrane (21) according to claim 38, wherein the blood purifier (10) is configured such that the square value of the correlation coefficient between the average value Pi, wherein i is an integer of not less than 3, of the transmembrane pressure differences and a solute leakage amount Ai during 240 minutes after the initiation of the blood purification is not less than 0.3.

40. The device for determining the transmembrane pressure difference across the blood purification membrane (21) according to claim 38, wherein the blood purifier (10) is configured such that the square value of the correlation coefficient between the transmembrane pressure difference Pj, wherein j is an integer of not less than 3, and a solute leakage concentration Nj at S = 240 minutes as a time 240 minutes after the initiation of the blood purification is not less than 0.4.

41. The device for determining the transmembrane pressure difference across the blood purification membrane (21) according to any one of claims 38 to 40, wherein the device is configured to calculate, from an actually measured value RNa(Tn) of the solute leakage concentration under the transmembrane pressure difference Pa(Tn) at each of the one or more predetermined times Tn after the initiation of the blood purification, a ratio between the target solute leakage concentration Na(Tn) and the actually measured value at each of the predetermined times Tn, to multiply the transmembrane pressure difference Pa(Tn) by the ratio to obtain a transmembrane pressure difference Pa'(Tn), and to determine the transmembrane pressure difference Pa'(Tn) to be the transmembrane pressure difference during the blood purification.

42. The device for determining the transmembrane pressure difference across the blood purification membrane (21) according to any one of claims 38 to 40, wherein the device is configured to change the transmembrane pressure difference Pa(Tn), while maintaining the average value P of the transmembrane pressure differences throughout entire blood purification treatment unchanged and preventing the supply flow rate of the replacement fluid at each of the predetermined times Tn from reaching an upper limit, to obtain a transmembrane pressure difference Pa"(Tn), and to determine the transmembrane pressure difference Pa"(Tn) to be the transmembrane pressure difference during the blood purification.

**Patentansprüche**

1. Blutreinigungsvorrichtung (1) zum Reinigen von Blut unter Verwendung eines Blutreinigers (10), wobei die Blutreinigungsvorrichtung (1) umfasst:

   eine Steuerungseinrichtung (15), die so konfiguriert ist, dass sie eine Transmembran-Druckdifferenz über eine Blutreinigungsmembran (21) des Blutreinigers (10) steuern kann,
   eine Funktionserfassungseinrichtung (90), die so konfiguriert ist, dass sie eine Funktion Fn erfassen kann, wobei die Funktionserfassungseinrichtung (90) umfasst:

   eine Erfassungseinrichtung (100), die so konfiguriert ist, dass sie jeweilige Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn) als Vielzahl von Transmembran-Druckdifferenzen Pm erfassen kann, wobei m eine ganze Zahl von nicht weniger als 2 ist; und
   eine Berechnungseinrichtung (92), die so konfiguriert ist, dass sie die Funktion Fn ausgehend von jeweiligen Werten der Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn), die von der Erfassungseinrichtung (100) erfasst wurden, erhalten kann,
   wobei auf der Basis der Funktion Fn, die durch den folgenden numerischen Ausdruck gegeben ist:

$$N(Tn) = Fn(P(Tn), Tn)$$

   wobei n eine ganze Zahl von nicht weniger als 1 ist, und
   wobei der numerische Ausdruck eine Beziehung zwischen der Transmembran-Druckdifferenz P(Tn) und der Solutdurchtrittskonzentration N(Tn) eines vorbestimmten Soluten im Blut an jedem des einen oder der mehreren vorbestimmten Zeitpunkte Tn nach der Einleitung der Blutreinigung zeigt, die Steuerungseinrichtung eine Transmembran-Druckdifferenz Pa(Tn), die einer Soll-Solutdurchtrittskonzentration Na(Tn) an jedem des einen oder der mehreren vorbestimmten Zeitpunkte Tn entspricht, erhält und die Transmembran-Druckdifferenz während der Blutreinigung auf der Basis der Transmembran-Druckdifferenz Pa(Tn) steuert.

2. Blutreinigungsvorrichtung (1) gemäß Anspruch 1, wobei die Funktionserfassungseinrichtung (90) Einrichtungen umfasst, die so konfiguriert sind, dass sie die Solutdurchtrittskonzentration N(Tn) erfassen können, wenn die Transmembran-Druckdifferenz P(Tn) für jeden des einen oder der mehreren vorbestimmten Zeitpunkte Tn nach der Einleitung der Blutreinigung geändert wird, wobei man die Funktion Fn erhält.

3. Blutreinigungsvorrichtung (1) gemäß einem der Ansprüche 1 oder 2, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

4. Blutreinigungsvorrichtung (1) gemäß einem der Ansprüche 1 bis 3, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

5. Blutreinigungsvorrichtung (1) gemäß einem der Ansprüche 1 bis 4, weiterhin umfassend:

   einen Blutkreislauf (11), der so konfiguriert ist, dass er das Blut dem Blutreiniger (10) zuführen/daraus ausleiten kann;
   einen Dialysatkreislauf (12), der so konfiguriert ist, dass er das Dialysat dem Blutreiniger (10) zuführen/daraus ausleiten kann; und
   einen Substituatkreislauf (13), der so konfiguriert ist, dass er auf einer Seite des Blutkreislaufes vor dem Blutreiniger (10) und/oder auf einer Seite des Blutkreislaufes hinter dem Blutreiniger (10) ein Substituat zuführen kann, wobei
die Steuerungseinrichtung (15) so konfiguriert ist, dass sie die Transmembran-Druckdifferenz steuern kann, indem sie die Zufuhrströmungsgeschwindigkeit des Substituats verändert.

6. Blutreinigungsvorrichtung (1) gemäß einem der Ansprüche 1 bis 5, wobei die Steuerungseinrichtung (15) so konfiguriert ist, dass sie die Transmembran-Druckdifferenz steuern kann, indem sie die Transmembran-Druckdifferenz stufenweise oder kontinuierlich verändert.

7. Blutreinigungsvorrichtung (1) gemäß einem der Ansprüche 1 bis 6, wobei die Steuerungseinrichtung (15) so konfiguriert ist, dass sie die Transmembran-Druckdifferenz regeln kann.

8. Blutreinigungsvorrichtung (1) gemäß einem der Ansprüche 1 bis 7, wobei die Steuerungseinrichtung (15) so konfiguriert ist, dass sie die Transmembran-Druckdifferenz während der Blutreinigung auf der Basis der Transmembran-Druckdifferenz $Pa(Tn)$ zu jedem der Zeitpunkte $Tn$ steuern kann, um die Solutdurchtrittskonzentrationen $N(Tn)$ während der Blutreinigung konstant zu halten.

9. Blutreinigungsvorrichtung (1) gemäß einem der Ansprüche 1 bis 8, weiterhin umfassend:
eine Schätzungseinrichtung (120), die so konfiguriert ist, dass sie ausgehend von Daten der Solutdurchtrittskonzentrationen $N1(Tn) ... Nm(Tn)$ unter der Vielzahl von Transmembran-Druckdifferenzen $P1(Tn) ... Pm(Tn)$ (wobei $m$ eine ganze Zahl von nicht weniger als 2 ist) an jedem des einen oder der mehreren vorbestimmten Zeitpunkte $Tn$ nach der Einleitung der Blutreinigung eine Datenkarte erzeugen kann, die die Solutdurchtrittskonzentrationen $N1(Tn) ... Nm(Tn)$ in einer Kombination von jedem der vorbestimmten Zeitpunkte $Tn$ und der Transmembran-Druckdifferenzen $P1(Tn) ... Pm(Tn)$ zeigt, und die Solutdurchtrittsmenge während der gesamten Blutreinigung oder pro Zeiteinheit anhand der Datenkarte und anhand der für jeden der Zeitpunkte eingestellten Transmembran-Druckdifferenz abschätzen kann.

10. Blutreinigungsvorrichtung (1) gemäß einem der Ansprüche 1 bis 8, weiterhin umfassend:
eine Schätzungseinrichtung (120), die so konfiguriert ist, dass sie die Solutdurchtrittsmenge während der gesamten Blutreinigung oder pro Zeiteinheit anhand der Funktion $Fn$ und anhand der für jeden der vorbestimmten Zeitpunkte eingestellten Soll-Transmembran-Druckdifferenz abschätzen kann.

11. Blutreinigungsvorrichtung (1) gemäß einem der Ansprüche 1 bis 10, wobei die Steuerungseinrichtung (15) so konfiguriert ist, dass sie anhand eines tatsächlich gemessenen Wertes $RNa(Tn)$ der Solutdurchtrittskonzentration unter der Transmembran-Druckdifferenz $Pa(Tn)$ an jedem des einen oder der mehreren vorbestimmten Zeitpunkte $Tn$ nach der Einleitung der Blutreinigung ein Verhältnis zwischen der Soll-Solutdurchtrittskonzentration $Na(Tn)$ und dem tatsächlich gemessenen Wert zu jedem der vorbestimmten Zeitpunkte $Tn$ berechnen kann, die Transmembran-Druckdifferenz $Pa(Tn)$ mit dem Verhältnis multiplizieren kann, wobei man eine Transmembran-Druckdifferenz $Pa'(Tn)$ erhält, und die Transmembran-Druckdifferenz während der Blutreinigung auf der Basis der Transmembran-Druckdifferenz $Pa'(Tn)$ steuern kann.

12. Blutreinigungsvorrichtung (1) gemäß einem der Ansprüche 1 bis 10, wobei die Steuerungseinrichtung (15) so konfiguriert ist, dass sie die Transmembran-Druckdifferenz $Pa(Tn)$ verändern kann, während der Mittelwert $P$ der Transmembran-Druckdifferenzen während der gesamten Blutreinigungsbehandlung unverändert gehalten wird und zu jedem der vorbestimmten Zeitpunkte $Tn$ verhindert wird, dass die Zufuhrströmungsgeschwindigkeit des Substituats eine Obergrenze erreicht, wobei man eine Transmembran-Druckdifferenz $Pa''(Tn)$ erhält, und die Transmembran-Druckdifferenz während der Blutreinigung auf der Basis der Transmembran-Druckdifferenz $Pa''(Tn)$ steuern kann.

13. Verfahren zum Erhalt einer Transmembran-Druckdifferenz über eine Blutreinigungsmembran (21), wenn Blut unter Verwendung eines Blutreinigers (10) gereinigt wird, umfassend die Schritte:
Erhalten einer Transmembran-Druckdifferenz $Pa(Tn)$, die an jedem des einen oder der mehreren vorbestimmten Zeitpunkte $Tn$ einer Soll-Solutdurchtrittskonzentration $Na(Tn)$ entspricht, aus einer Funktion $Fn$, die durch einen numerischen Ausdruck gegeben ist:

$N(Tn) = Fn(P(Tn),Tn)$ wobei $n$ eine ganze Zahl von nicht weniger als 1 ist,
wobei der numerische Ausdruck eine Beziehung zwischen einer Transmembran-Druckdifferenz $P(Tn)$ und einer Solutdurchtrittskonzentration $N(Tn)$ eines vorbestimmten Soluten im Blut an jedem des einen oder der mehreren vorbestimmten Zeitpunkte $Tn$ nach der Einleitung der Blutreinigung zeigt,

wobei die Funktion Fn durch eine Funktionserfassungseinrichtung (90) erfasst wird,
wobei die Funktionserfassungseinrichtung (90) umfasst:

eine Erfassungseinrichtung (100), die so konfiguriert ist, dass sie jeweilige Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn) als Vielzahl von Transmembran-Druckdifferenzen Pm erfassen kann, wobei m eine ganze Zahl von nicht weniger als 2 ist; und
eine Berechnungseinrichtung (92), die so konfiguriert ist, dass sie die Funktion Fn ausgehend von jeweiligen Werten der Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn), die von der Erfassungseinrichtung (100) erfasst wurden, erhalten kann,
mit der Maßgabe, dass Verfahren zur Behandlung des menschlichen oder tierischen Körpers durch Therapie und Chirurgie ausgeschlossen sind.

14. Verfahren zum Erhalt der Transmembran-Druckdifferenz über die Blutreinigungsmembran (21) gemäß Anspruch 13, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

15. Verfahren zum Erhalt der Transmembran-Druckdifferenz über die Blutreinigungsmembran (21) gemäß Anspruch 13, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

16. Verfahren zum Erhalt der Transmembran-Druckdifferenz über die Blutreinigungsmembran (21) gemäß einem der Ansprüche 13 bis 15, wobei das Verfahren anhand eines tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnet und die Transmembran-Druckdifferenz Pa(Tn) mit dem Verhältnis multipliziert, wobei man eine Transmembran-Druckdifferenz Pa'(Tn) erhält.

17. Verfahren zum Erhalt der Transmembran-Druckdifferenz über die Blutreinigungsmembran (21) gemäß einem der Ansprüche 13 bis 15, wobei das Verfahren die Transmembran-Druckdifferenz Pa(Tn) verändert, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält.

18. Verfahren zum Erhalt der Transmembran-Druckdifferenz über die Blutreinigungsmembran (21) gemäß Anspruch 13, weiterhin umfassend den Schritt des Bestimmens einer Transmembran-Druckdifferenz während der Blutreinigung auf der Basis der Transmembran-Druckdifferenz Pa(Tn).

19. Verfahren zur Bestimmung der Transmembran-Druckdifferenz über die Blutreinigungsmembran (21) gemäß Anspruch 18, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

20. Verfahren zur Bestimmung der Transmembran-Druckdifferenz über die Blutreinigungsmembran (21) gemäß Anspruch 18, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

21. Verfahren zur Bestimmung der Transmembran-Druckdifferenz über die Blutreinigungsmembran (21) gemäß einem der Ansprüche 18 bis 20, wobei das Verfahren anhand eines tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnet, die Transmembran-Druckdifferenz Pa(Tn) mit dem Verhältnis multipliziert, wobei man eine Transmembran-Druckdifferenz Pa'(Tn) erhält, und die Transmembran-Druckdifferenz Pa'(Tn) als Transmembran-Druckdifferenz während der Blutreinigung bestimmt.

22. Verfahren zur Bestimmung der Transmembran-Druckdifferenz über die Blutreinigungsmembran (21) gemäß einem der Ansprüche 18 bis 20, wobei das Verfahren die Transmembran-Druckdifferenz Pa(Tn) verändert, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält, und die Transmembran-Druckdifferenz Pa"(Tn) als Transmembran-Druckdifferenz während der Blutreinigung bestimmt.

23. Vorrichtung zum Erhalt einer Transmembran-Druckdifferenz über eine Blutreinigungsmembran (21), wenn Blut unter Verwendung eines Blutreinigers (10) gereinigt wird, wobei die Vorrichtung aus einer Funktion Fn, die durch einen numerischen Ausdruck gegeben ist:

$$N(Tn) = Fn(P(Tn),Tn)$$

wobei n eine ganze Zahl von nicht weniger als 1 ist,
wobei der numerische Ausdruck eine Beziehung zwischen einer Transmembran-Druckdifferenz P(Tn) und einer Solutdurchtrittskonzentration N(Tn) eines vorbestimmten Soluten im Blut an jedem des einen oder der mehreren vorbestimmten Zeitpunkte Tn nach der Einleitung der Blutreinigung zeigt, eine Transmembran-Druckdifferenz Pa(Tn) erhält, die an jedem des einen oder der mehreren vorbestimmten Zeitpunkte Tn einer Soll-Solutdurchtrittskonzentration Na(Tn) entspricht,
wobei die Funktion Fn durch eine Funktionserfassungseinrichtung (90) erfasst wird,
wobei
die Funktionserfassungseinrichtung (90) umfasst:

eine Erfassungseinrichtung (100), die so konfiguriert ist, dass sie jeweilige Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn) als Vielzahl von Transmembran-Druckdifferenzen Pm erfassen kann, wobei m eine ganze Zahl von nicht weniger als 2 ist; und
eine Berechnungseinrichtung (92), die so konfiguriert ist, dass sie die Funktion Fn ausgehend von jeweiligen Werten der Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn), die von der Erfassungseinrichtung (100) erfasst wurden, erhalten kann.

24. Vorrichtung zum Erhalt der Transmembran-Druckdifferenz über die Blutreinigungsmembran (21) gemäß Anspruch 23, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

25. Vorrichtung zum Erhalt der Transmembran-Druckdifferenz über die Blutreinigungsmembran (21) gemäß Anspruch 23, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

26. Vorrichtung zum Erhalt der Transmembran-Druckdifferenz über die Blutreinigungsmembran (21) gemäß einem der Ansprüche 23 bis 25, wobei die Vorrichtung so konfiguriert ist, dass sie anhand des tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnen kann und die Transmembran-Druckdifferenz Pa(Tn) mit dem Verhältnis multiplizieren kann, wobei man eine Transmembran-Druckdifferenz Pa'(Tn) erhält.

27. Vorrichtung zum Erhalt der Transmembran-Druckdifferenz über die Blutreinigungsmembran (21) gemäß einem der Ansprüche 23 bis 26, wobei die Vorrichtung so konfiguriert ist, dass sie die Transmembran-Druckdifferenz Pa(Tn) verändern kann, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält.

28. Programm zum Bewirken, dass ein Computer (15) ein Verfahren zum Erhalt einer Transmembran-Druckdifferenz über eine Blutreinigungsmembran (21), wenn Blut unter Verwendung eines Blutreinigers (10) gereinigt wird, implementiert, wobei das Verfahren den folgenden Schritt umfasst:
Erhalten einer Transmembran-Druckdifferenz Pa(Tn), die an jedem des einen oder der mehreren vorbestimmten Zeitpunkte Tn einer Soll-Solutdurchtrittskonzentration Na(Tn) entspricht, aus einer Funktion Fn, die durch einen numerischen Ausdruck gegeben ist:

N(Tn) = Fn(P(Tn),Tn) wobei n eine ganze Zahl von nicht weniger als 1 ist,
wobei der numerische Ausdruck eine Beziehung zwischen einer Transmembran-Druckdifferenz P(Tn) und einer Solutdurchtrittskonzentration N(Tn) eines vorbestimmten Soluten im Blut an jedem des einen oder der mehreren vorbestimmten Zeitpunkte Tn nach der Einleitung der Blutreinigung zeigt,
wobei die Funktion Fn durch eine Funktionserfassungseinrichtung (90) erfasst wird, wobei
die Funktionserfassungseinrichtung (90) umfasst:

eine Erfassungseinrichtung (100), die so konfiguriert ist, dass sie jeweilige Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn) als Vielzahl von Transmembran-Druckdifferenzen Pm (wobei m eine ganze Zahl von nicht weniger als 2 ist) erfassen kann; und
eine Berechnungseinrichtung (92), die so konfiguriert ist, dass sie die Funktion Fn ausgehend von jeweiligen Werten der Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn), die von der Erfassungseinrichtung (100) erfasst wurden, erhalten kann.

29. Programm gemäß Anspruch 28, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

30. Programm gemäß Anspruch 28, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

31. Programm gemäß einem der Ansprüche 28 bis 30, wobei das Programm anhand eines tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnet und die Transmembran-Druckdifferenz Pa(Tn) mit dem Verhältnis multipliziert, wobei man eine Transmembran-Druckdifferenz Pa'(Tn) erhält.

32. Programm gemäß einem der Ansprüche 28 bis 30, wobei das Programm die Transmembran-Druckdifferenz Pa(Tn) verändert, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält.

33. Programm gemäß Anspruch 28, weiterhin umfassend den Schritt des Bestimmens einer Transmembran-Druckdifferenz während der Blutreinigung auf der Basis der Transmembran-Druckdifferenz Pa(Tn).

34. Programm gemäß Anspruch 33, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

35. Programm gemäß Anspruch 33, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

36. Programm gemäß einem der Ansprüche 33 bis 35, wobei das Programm anhand eines tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnet, die Transmembran-Druckdifferenz Pa(Tn) mit dem Verhältnis multipliziert, wobei man eine Transmembran-Druckdifferenz Pa'(Tn) erhält, und die Transmembran-Druckdifferenz Pa'(Tn) als Transmembran-Druckdifferenz während der Blutreinigung bestimmt.

37. Programm gemäß einem der Ansprüche 33 bis 35, wobei das Programm die Transmembran-Druckdifferenz Pa(Tn) verändert, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält, und die Transmembran-Druckdifferenz Pa"(Tn) als Transmembran-Druckdifferenz während der Blutreinigung bestimmt.

38. Vorrichtung zur Bestimmung einer Transmembran-Druckdifferenz über eine Blutreinigungsmembran (21), wenn Blut unter Verwendung eines Blutreinigers (10) gereinigt wird, wobei die Vorrichtung eine Transmembran-Druckdifferenz Pa(Tn), die an jedem des einen oder der mehreren vorbestimmten Zeitpunkte Tn einer Soll-Solutdurchtrittskonzentration Na(Tn) entspricht, auf der Basis einer Funktion Fn erhält, die durch einen numerischen Ausdruck gegeben ist:

N(Tn) = Fn(P(Tn),Tn) wobei n eine ganze Zahl von nicht weniger als 1 ist,
wobei der numerische Ausdruck eine Beziehung zwischen einer Transmembran-Druckdifferenz P(Tn) und einer Solutdurchtrittskonzentration N(Tn) eines vorbestimmten Soluten im Blut an jedem des einen oder der mehreren vorbestimmten Zeitpunkte Tn nach der Einleitung der Blutreinigung zeigt, und eine Transmembran-Druckdifferenz während der Blutreinigung auf der Basis der Transmembran-Druckdifferenz Pa(Tn) bestimmt,
wobei die Funktion Fn durch eine Funktionserfassungseinrichtung (90) erfasst wird, wobei die Funktionserfassungseinrichtung (90) umfasst:

eine Erfassungseinrichtung (100), die so konfiguriert ist, dass sie jeweilige Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn) als Vielzahl von Transmembran-Druckdifferenzen Pm (wobei m eine ganze Zahl von nicht weniger als 2 ist) erfassen kann; und eine Berechnungseinrichtung (92), die so konfiguriert ist, dass sie die Funktion Fn ausgehend von jeweiligen Werten der Solutdurchtrittskonzentrationen N1(Tn) ... Nm(Tn) an jedem des einen oder der mehreren vorbestimmten Zeitpunkte Tn für die jeweiligen Transmembran-Druckdifferenzen P1(Tn) ... Pm(Tn), die von der Erfassungseinrichtung (100) erfasst wurden, erhalten kann.

39. Vorrichtung zur Bestimmung der Transmembran-Druckdifferenz über die Blutreinigungsmembran (21) gemäß Anspruch 38, wobei der Blutreiniger (10) so konfiguriert ist, dass das Quadrat des Korrelationskoeffizienten zwischen dem Mittelwert Pi, wobei i eine ganze Zahl von nicht weniger als 3 ist, der Transmembran-Druckdifferenzen und der Solutdurchtrittsmenge Ai während 240 Minuten nach der Einleitung der Blutreinigung nicht weniger als 0,3 beträgt.

40. Vorrichtung zur Bestimmung der Transmembran-Druckdifferenz über die Blutreinigungsmembran (21) gemäß Anspruch 38, wobei der Blutreiniger (10) so konfiguriert ist, dass das Quadrat des Korrelationskoeffizienten zwischen der Transmembran-Druckdifferenz Pj, wobei j eine ganze Zahl von nicht weniger als 3 ist, und der Solutdurchtrittskonzentration Nj bei S = 240 Minuten als Zeitpunkt 240 Minuten nach der Einleitung der Blutreinigung nicht weniger als 0,4 beträgt.

**41.** Vorrichtung zur Bestimmung der Transmembran-Druckdifferenz über die Blutreinigungsmembran (21) gemäß einem der Ansprüche 38 bis 40, wobei die Vorrichtung so konfiguriert ist, dass sie anhand eines tatsächlich gemessenen Wertes RNa(Tn) der Solutdurchtrittskonzentration unter der Transmembran-Druckdifferenz Pa(Tn) an jedem des einen oder der mehreren vorbestimmten Zeitpunkte Tn nach der Einleitung der Blutreinigung ein Verhältnis zwischen der Soll-Solutdurchtrittskonzentration Na(Tn) und dem tatsächlich gemessenen Wert zu jedem der vorbestimmten Zeitpunkte Tn berechnen kann, die Transmembran-Druckdifferenz Pa(Tn) mit dem Verhältnis multiplizieren kann, wobei man eine Transmembran-Druckdifferenz Pa'(Tn) erhält, und die Transmembran-Druckdifferenz Pa'(Tn) als Transmembran-Druckdifferenz während der Blutreinigung bestimmen kann.

**42.** Vorrichtung zur Bestimmung der Transmembran-Druckdifferenz über die Blutreinigungsmembran (21) gemäß einem der Ansprüche 38 bis 40, wobei die Vorrichtung so konfiguriert ist, dass sie die Transmembran-Druckdifferenz Pa(Tn) verändern kann, während der Mittelwert P der Transmembran-Druckdifferenzen während der gesamten Blutreinigungsbehandlung unverändert gehalten wird und zu jedem der vorbestimmten Zeitpunkte Tn verhindert wird, dass die Zufuhrströmungsgeschwindigkeit des Substituats eine Obergrenze erreicht, wobei man eine Transmembran-Druckdifferenz Pa"(Tn) erhält, und die Transmembran-Druckdifferenz Pa"(Tn) als Transmembran-Druckdifferenz während der Blutreinigung bestimmen kann.

**Revendications**

**1.** Dispositif de purification du sang (1) pour purifier du sang à l'aide d'un purificateur de sang (10), le dispositif de purification du sang (1) comprenant:

un moyen de commande (15) configuré pour commander une différence de pression transmembranaire à travers une membrane de purification du sang (21) du purificateur de sang (10),
un moyen d'acquisition de fonction (90) configuré pour acquérir une fonction Fn, où le moyen d'acquisition de fonction (90) inclut:

un moyen d'acquisition (100) configuré pour acquérir des concentrations de fuite de soluté respectives N1(Tn) ... Nm(Tn) à chacun des un ou plusieurs instants prédéterminés Tn pour les différences de pression transmembranaire respectives P1(Tn) ... Pm(Tn) sous forme d'une pluralité de différences de pression transmembranaire Pm, où m est un nombre entier non inférieur à 2; et
un moyen de calcul (92) configuré pour obtenir la fonction Fn à partir de valeurs respectives des concentrations de fuite de soluté N1(Tn) ... Nm(Tn) à chacun des un ou plusieurs instants prédéterminés Tn pour les différences de pression transmembranaires respectives P1(Tn) ... Pm(Tn) acquises par le moyen d'acquisition (100),
où sur la base de la fonction Fn donnée par l'expression numérique:

$$N(Tn) = Fn(P(Tn), Tn)$$

où n est un nombre entier non inférieur à 1, et
où l'expression numérique montre une relation entre la différence de pression transmembranaire P(Tn) et la concentration de fuite de soluté N(Tn) d'un soluté prédéterminé dans le sang à chacun des un ou plusieurs instants prédéterminés Tn après le début de la purification du sang, le moyen de commande obtient une différence de pression transmembranaire Pa(Tn) correspondant à une concentration de fuite de soluté cible Na(Tn) à chacun des un ou plusieurs instants prédéterminés Tn, et commande la différence de pression transmembranaire pendant la purification du sang sur la base de la différence de pression transmembranaire Pa(Tn).

**2.** Dispositif de purification du sang (1) selon la revendication 1, où le moyen d'acquisition de fonction (90) inclut un moyen configuré pour acquérir la concentration de fuite de soluté N(Tn) lorsque la différence de pression transmembranaire P(Tn) est modifiée pour chacun des un ou plusieurs instants prédéterminés Tn après le début de la purification du sang pour obtenir la fonction Fn.

**3.** Dispositif de purification du sang (1) selon l'une quelconque des revendications 1 ou 2, où le purificateur de sang (10) est configuré de sorte que la valeur quadratique du coefficient de corrélation entre la valeur moyenne Pi, où i est un nombre entier non inférieur à 3, des différences de pression transmembranaire et la quantité de fuite de soluté Ai pendant 240 minutes après le début de la purification du sang n'est pas inférieure à 0,3.

4.  Dispositif de purification du sang (1) selon l'une quelconque des revendications 1 à 3, où le purificateur de sang (10) est configuré de sorte que la valeur quadratique du coefficient de corrélation entre la différence de pression transmembranaire Pj, où j est un nombre entier non inférieur à 3, et la concentration de fuite de soluté Nj à S = 240 minutes comme instant 240 minutes après le début de la purification du sang n'est pas inférieure à 0,4.

5.  Dispositif de purification du sang (1) selon l'une quelconque des revendications 1 à 4, comprenant en outre:

    un circuit de sang (11) configuré pour fournir/évacuer le sang au/depuis le purificateur de sang (10);
    un circuit de dialysat (12) configuré pour fournir/évacuer le dialysat au/depuis le purificateur de sang (10); et
    un circuit de fluide de remplacement (13) configuré pour fournir un fluide de remplacement à un côté du circuit de sang en amont du purificateur de sang (10) et/ou à un côté du circuit de sang en aval du purificateur de sang (10), où
    le moyen de commande (15) est configuré pour commander la différence de pression transmembranaire en modifiant un débit de fourniture du fluide de remplacement.

6.  Dispositif de purification du sang (1) selon l'une quelconque des revendications 1 à 5, où le moyen de commande (15) est configuré pour commander la différence de pression transmembranaire en modifiant progressivement ou en continu la différence de pression transmembranaire.

7.  Dispositif de purification du sang (1) selon l'une quelconque des revendications 1 à 6, où le moyen de commande (15) est configuré pour commander la différence de pression transmembranaire par une commande de rétroaction.

8.  Dispositif de purification du sang (1) selon l'une quelconque des revendications 1 à 7, où le moyen de commande (15) est configuré pour commander la différence de pression transmembranaire pendant la purification du sang sur la base de la différence de pression transmembranaire Pa(Tn) à chacun des instants Tn de manière à maintenir constante la concentration de fuite de soluté N(Tn) pendant la purification du sang.

9.  Dispositif de purification du sang (1) selon l'une quelconque des revendications 1 à 8, comprenant en outre:
    un moyen d'estimation (120) configuré pour produire, à partir de données des concentrations de fuite de soluté N1(Tn) ... Nm(Tn) sous la pluralité de différences de pression transmembranaire P1(Tn) ... Pm(Tn) (où m est un nombre entier non inférieur à 2) à chacun des un ou plusieurs instants prédéterminés Tn après le début de la purification du sang, une carte de données montrant les concentrations de fuite de soluté N1(Tn) ... Nm(Tn) dans une combinaison de chacun des instants prédéterminés Tn et des différences de pression transmembranaire P1(Tn) ... Pm(Tn), et pour estimer la quantité de fuite de soluté pendant toute la purification du sang ou par instant unitaire à partir de la carte de données et à partir de la différence de pression transmembranaire établie pour chacun des instants.

10. Dispositif de purification du sang (1) selon l'une quelconque des revendications 1 à 8, comprenant en outre:
    un moyen d'estimation (120) configuré pour estimer la quantité de fuite de soluté pendant toute la purification du sang ou par instant unitaire à partir de la fonction Fn et à partir d'une différence de pression transmembranaire cible établie pour chacun des instants prédéterminés.

11. Dispositif de purification du sang (1) selon l'une quelconque des revendications 1 à 10, où le moyen de commande (15) est configuré pour calculer, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, un rapport entre la concentration de fuite de soluté cible Na(Tn) et la valeur mesurée réellement à chacun des instants prédéterminés Tn, pour multiplier la différence de pression transmembranaire Pa(Tn) par le rapport pour obtenir une différence de pression transmembranaire Pa'(Tn), et pour commander la différence de pression transmembranaire pendant la purification du sang sur la base de la différence de pression transmembranaire Pa'(Tn).

12. Dispositif de purification du sang (1) selon l'une quelconque des revendications 1 à 10, où le moyen de commande (15) est configuré pour modifier la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn), et pour commander la différence de pression transmembranaire pendant la purification du sang sur la base de la différence de pression transmembranaire Pa"(Tn).

13. Procédé pour obtenir une différence de pression transmembranaire à travers une membrane de purification du sang (21), lorsque du sang est purifié à l'aide d'un purificateur de sang (10), qui comprend les étapes de:
obtention, à partir d'une fonction Fn donnée par une expression numérique:

N(Tn) = Fn(P(Tn), Tn), où n est un nombre entier non inférieur à 1
où l'expression numérique montre une relation entre une différence de pression transmembranaire P(Tn) et une concentration de fuite de soluté N(Tn) d'un soluté prédéterminé dans le sang à chacun de un ou plusieurs instants prédéterminés Tn après le début de la purification du sang, une différence de pression transmembranaire Pa(Tn) correspondant à une concentration de fuite de soluté cible Na(Tn) à chacun des un ou plusieurs instants prédéterminés Tn,
où la fonction Fn est acquise par un moyen d'acquisition de fonction (90),
où le moyen d'acquisition de fonction (90) inclut:

un moyen d'acquisition (100) configuré pour acquérir des concentrations de fuite de soluté respectives N1(Tn) ... Nm(Tn) à chacun des un ou plusieurs instants prédéterminés Tn pour des différences de pression transmembranaire respectives P1(Tn) ... Pm(Tn) sous forme d'une pluralité de différences de pression transmembranaire Pm, où m est un nombre entier non inférieur à 2; et
un moyen de calcul (92) configuré pour obtenir la fonction Fn à partir de valeurs respectives des concentrations de fuite de soluté N 1 (Tn) ... Nm(Tn) à chacun des un ou plusieurs instants prédéterminés Tn pour les différences de pression transmembranaire respectives P1(Tn) ... Pm(Tn) acquises par le moyen d'acquisition (100),
à condition que les procédés pour le traitement du corps humain et animal par thérapie et chirurgie soient exclus.

14. Procédé pour obtenir la différence de pression transmembranaire à travers la membrane de purification du sang (21) selon la revendication 13, où le purificateur de sang (10) est configuré de sorte que la valeur quadratique du coefficient de corrélation entre la valeur moyenne Pi, où i est un nombre entier non inférieur à 3, des différences de pression transmembranaire et la quantité de fuite de soluté Ai pendant 240 minutes après le début de la purification du sang n'est pas inférieure à 0,3.

15. Procédé pour obtenir la différence de pression transmembranaire à travers la membrane de purification du sang (21) selon la revendication 13, où le purificateur de sang (10) est configuré de sorte que la valeur quadratique du coefficient de corrélation entre la différence de pression transmembranaire Pj, où j est un nombre entier non inférieur à 3, et la concentration de fuite de soluté Nj à S = 240 minutes comme instant 240 minutes après le début de la purification du sang n'est pas inférieure à 0,4.

16. Procédé pour obtenir la différence de pression transmembranaire à travers la membrane de purification du sang (21) selon l'une quelconque des revendications 13 à 15, où le procédé calcule, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, le rapport entre la concentration de fuite de soluté cible Na(Tn) et la valeur mesurée réellement à chacun des instants prédéterminés Tn, et multiplie la différence de pression transmembranaire Pa(Tn) par le rapport pour obtenir une différence de pression transmembranaire Pa'(Tn).

17. Procédé pour obtenir la différence de pression transmembranaire à travers la membrane de purification du sang (21) selon l'une quelconque des revendications 13 à 15, où le procédé modifie la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn).

18. Procédé pour obtenir la différence de pression transmembranaire à travers la membrane de purification du sang (21) selon la revendication 13, comprenant en outre l'étape de détermination d'une différence de pression transmembranaire pendant la purification du sang sur la base de la différence de pression transmembranaire Pa(Tn).

**19.** Procédé pour déterminer la différence de pression transmembranaire à travers la membrane de purification du sang (21) selon la revendication 18, où le purificateur de sang (10) est configuré de sorte que la valeur quadratique du coefficient de corrélation entre la valeur moyenne Pi, où i est un nombre entier non inférieur à 3, des différences de pression transmembranaire et la quantité de fuite de soluté Ai pendant 240 minutes après le début de la purification du sang n'est pas inférieure à 0,3.

**20.** Procédé pour déterminer la différence de pression transmembranaire à travers la membrane de purification du sang (21) selon la revendication 18, où le purificateur de sang (10) est configuré de sorte que la valeur quadratique du coefficient de corrélation entre la différence de pression transmembranaire Pj, où j est un nombre entier non inférieur à 3, et la concentration de fuite de soluté Nj à S = 240 minutes comme instant 240 minutes après le début de la purification du sang n'est pas inférieure à 0,4.

**21.** Procédé pour déterminer la différence de pression transmembranaire à travers la membrane de purification du sang (21) selon l'une quelconque des revendications 18 à 20, où le procédé calcule, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, un rapport entre la concentration de fuite de soluté cible Na(Tn) et la valeur mesurée réellement à chacun des instants prédéterminés Tn, multiplie la différence de pression transmembranaire Pa(Tn) par le rapport pour obtenir une différence de pression transmembranaire Pa'(Tn), et détermine la différence de pression transmembranaire Pa'(Tn) comme étant la différence de pression transmembranaire pendant la purification du sang.

**22.** Procédé pour déterminer la différence de pression transmembranaire à travers la membrane de purification du sang (21) selon l'une quelconque des revendications 18 à 20, où le procédé modifie la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn), et détermine la différence de pression transmembranaire Pa"(Tn) comme étant la différence de pression transmembranaire pendant la purification du sang.

**23.** Dispositif pour obtenir une différence de pression transmembranaire à travers une membrane de purification du sang (21) lorsque du sang est purifié à l'aide d'un purificateur de sang (10), où le dispositif obtient, à partir d'une fonction Fn donnée par une expression numérique:

N(Tn) = Fn(P(Tn), Tn), où n est un nombre entier non inférieur à 1,
où l'expression numérique montre une relation entre une différence de pression transmembranaire P(Tn) et une concentration de fuite de soluté N(Tn) d'un soluté prédéterminé dans le sang à chacun de un ou plusieurs instants prédéterminés Tn après le début de la purification du sang, une différence de pression transmembranaire Pa(Tn) correspondant à une concentration de fuite de soluté cible Na(Tn) à chacun des un ou plusieurs instants prédéterminés Tn, où la fonction Fn est acquise par un moyen d'acquisition de fonction (90),
où
le moyen d'acquisition de fonction (90) inclut:

un moyen d'acquisition (100) configuré pour acquérir des concentrations de fuite de soluté respectives N1(Tn) ... Nm(Tn) à chacun des un ou plusieurs instants prédéterminés Tn pour des différences de pression transmembranaire respectives P1(Tn) ... Pm (Tn) sous forme d'une pluralité de différences de pression transmembranaire Pm, où m est un nombre entier non inférieur à 2; et
un moyen de calcul (92) configuré pour obtenir la fonction Fn à partir de valeurs respectives des concentrations de fuite de soluté N 1 (Tn) ... Nm(Tn) à chacun des un ou plusieurs instants prédéterminés Tn pour les différences de pression transmembranaires respectives P1(Tn) ... Pm(Tn) acquises par le moyen d'acquisition (100).

**24.** Dispositif pour obtenir la différence de pression transmembranaire à travers la membrane de purification du sang (21) selon la revendication 23, où le purificateur de sang (10) est configuré de sorte que la valeur quadratique du coefficient de corrélation entre la valeur moyenne Pi, où i est un nombre entier non inférieur à 3, des différences de pression transmembranaire et la quantité de fuite de soluté Ai pendant 240 minutes après le début de la purification du sang n'est pas inférieure à 0,3.

**25.** Dispositif pour obtenir la différence de pression transmembranaire à travers la membrane de purification du sang (21) selon la revendication 23, où le purificateur de sang (10) est configuré de sorte que la valeur quadratique du coefficient de corrélation entre la différence de pression transmembranaire Pj, où j est un nombre entier non inférieur à 3, et la concentration de fuite de soluté Nj à S = 240 minutes comme instant 240 minutes après le début de la purification du sang n'est pas inférieure à 0,4.

26. Dispositif pour obtenir la différence de pression transmembranaire à travers la membrane de purification du sang (21) selon l'une quelconque des revendications 23 à 25, où le dispositif est configuré pour calculer, à partir de la valeur mesurée réellement RNa(Tn) de la concentration de fuite de soluté sous la différence de pression transmembranaire Pa(Tn) à chacun des un ou plusieurs instants prédéterminés Tn après le début de la purification du sang, le rapport entre la concentration de fuite de soluté cible Na(Tn) et la valeur mesurée réellement à chacun des instants prédéterminés Tn, et pour multiplier la différence de pression transmembranaire Pa(Tn) par le rapport pour obtenir une différence de pression transmembranaire Pa'(Tn).

27. Dispositif pour obtenir la différence de pression transmembranaire à travers la membrane de purification du sang (21) selon l'une quelconque des revendications 23 à 26, où le dispositif est configuré pour modifier la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn).

28. Programme pour amener un ordinateur (15) à mettre en oeuvre un procédé pour obtenir une différence de pression transmembranaire à travers une membrane de purification du sang lorsque du sang est purifié à l'aide d'un purificateur de sang (10), le procédé comprenant une étape de:

obtention, à partir d'une fonction Fn donnée par une expression numérique:

N(Tn) = Fn(P(Tn), Tn), où n est un nombre entier non inférieur à 1,

où l'expression numérique montre une relation entre une différence de pression transmembranaire P(Tn) et une concentration de fuite de soluté N(Tn) d'un soluté prédéterminé dans le sang à chacun de un ou plusieurs instants prédéterminés Tn après le début de la purification du sang, une différence de pression transmembranaire Pa(Tn) correspondant à une concentration de fuite de soluté cible Na(Tn) à chacun des un ou plusieurs instants prédéterminés Tn,

où la fonction Fn est acquise par un moyen d'acquisition de fonction (90), où

le moyen d'acquisition de fonction (90) inclut:

un moyen d'acquisition (100) configuré pour acquérir des concentrations de fuite de soluté respectives N1(Tn) ... Nm(Tn) à chacun des un ou plusieurs instants prédéterminés Tn pour des différences de pression transmembranaire respectives P1(Tn) ... Pm(Tn) sous forme d'une pluralité de différences de pression transmembranaire Pm (où m est un nombre entier non inférieur à 2 ); et

un moyen de calcul (92) configuré pour obtenir la fonction Fn à partir de valeurs respectives des concentrations de fuite de soluté N1(Tn) ... Nm (Tn) à chacun des un ou plusieurs instants prédéterminés Tn pour les différences de pression transmembranaires respectives P1(Tn) ... Pm(Tn) acquises par le moyen d'acquisition (100).

29. Programme selon la revendication 28, où le purificateur de sang (10) est configuré de sorte que la valeur quadratique du coefficient de corrélation entre la valeur moyenne Pi, où i est un nombre entier non inférieur à 3, des différences de pression transmembranaire et une quantité de fuite de soluté Ai pendant 240 minutes après le début de la purification du sang n'est pas inférieure à 0,3.

30. Programme selon la revendication 28, où, le purificateur de sang (10) configuré de sorte que la valeur quadratique du coefficient de corrélation entre la différence de pression transmembranaire Pj, où j est un nombre entier non inférieur à 3, et une concentration de fuite de soluté Nj à S = 240 minutes comme instant 240 minutes après le début de la purification du sang n'est pas inférieure à 0,4.

31. Programme selon l'une quelconque des revendications 28 à 30, où le programme calcule, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, un rapport entre la concentration de fuite de soluté cible Na(Tn) et la valeur mesurée réellement à chacun des instants prédéterminés Tn, et multiplie la différence de pression transmembranaire Pa(Tn) par le rapport pour obtenir une différence de pression transmembranaire Pa'(Tn).

32. Programme selon l'une quelconque des revendications 28 à 30, où le programme modifie la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn).

33. Programme selon la revendication 28 comprenant en outre l'étape de détermination d'une différence de pression transmembranaire pendant la purification du sang sur la base de la différence de pression transmembranaire Pa(Tn).

34. Programme selon la revendication 33, où le purificateur de sang (10) est configuré de sorte que la valeur quadratique du coefficient de corrélation entre la valeur moyenne Pi, où i est un nombre entier non inférieur à 3, des différences de pression transmembranaire et la quantité de fuite de soluté Ai pendant 240 minutes après le début de la purification du sang n'est pas inférieure à 0,3.

35. Programme selon la revendication 33, où le purificateur de sang (10) est configuré de sorte que la valeur quadratique du coefficient de corrélation entre la différence de pression transmembranaire Pj, où j est un nombre entier non inférieur à 3, et une concentration de fuite de soluté Nj à S = 240 minutes comme instant 240 minutes après le début de la purification du sang n'est pas inférieure à 0,4.

36. Programme selon l'une quelconque des revendications 33 à 35, où le programme calcule, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, un rapport entre la concentration de fuite de soluté cible Na(Tn) et la valeur mesurée réellement à chacun des instants prédéterminés Tn, multiplie la différence de pression transmembranaire Pa(Tn) par le rapport pour obtenir une différence de pression transmembranaire Pa'(Tn), et détermine la différence de pression transmembranaire Pa'(Tn) comme étant la différence de pression transmembranaire pendant la purification du sang.

37. Programme selon l'une quelconque des revendications 33 à 35, où le programme modifie la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn), et détermine la différence de pression transmembranaire Pa"(Tn) comme étant la différence de pression transmembranaire pendant la purification du sang.

38. Dispositif pour déterminer une différence de pression transmembranaire à travers une membrane de purification du sang (21) lorsque du sang est purifié à l'aide d'un purificateur de sang (10), où le dispositif obtient, sur la base d'une fonction Fn donnée par une expression numérique :

N(Tn) = Fn(P(Tn), Tn) où n est un nombre entier non inférieur à 1,
où l'expression numérique montre une relation entre une différence de pression transmembranaire P(Tn) et une concentration de fuite de soluté N(Tn) d'un soluté prédéterminé dans le sang à chacun de un ou plusieurs instants prédéterminés Tn après le début de la purification du sang, une différence de pression transmembranaire Pa(Tn) correspondant à une concentration de fuite de soluté cible Na(Tn) à chacun des un ou plusieurs instants prédéterminés Tn, et détermine une différence de pression transmembranaire pendant la purification du sang sur la base de la différence de pression transmembranaire Pa(Tn),
où la fonction Fn est acquise par un moyen d'acquisition de fonction (90), où
le moyen d'acquisition de fonction (90) inclut :

un moyen d'acquisition (100) configuré pour acquérir des concentrations de fuite de soluté respectives N1(Tn) ... Nm(Tn) à chacun des un ou plusieurs instants prédéterminés Tn pour des différences de pression transmembranaire respectives P1(Tn) ... Pm(Tn) sous forme d'une pluralité de différences de pression transmembranaire Pm (où m est un nombre entier non inférieur à 2 ); et
un moyen de calcul (92) configuré pour obtenir la fonction Fn à partir de valeurs respectives des concentrations de fuite de soluté N 1 (Tn) ... Nm(Tn) à chacun des un ou plusieurs instants prédéterminés Tn pour les différences de pression transmembranaire respectives P1(Tn) ... Pm(Tn) acquises par le moyen d'acquisition (100).

**39.** Dispositif pour déterminer la différence de pression transmembranaire à travers la membrane de purification du sang (21) selon la revendication 38, où le purificateur de sang (10) est configuré de sorte que la valeur quadratique du coefficient de corrélation entre la valeur moyenne Pi, où i est un nombre entier non inférieur à 3, des différences de pression transmembranaire et une quantité de fuite de soluté Ai pendant 240 minutes après le début de la purification du sang n'est pas inférieure à 0,3.

**40.** Dispositif pour déterminer la différence de pression transmembranaire à travers la membrane de purification du sang (21) selon la revendication 38, où le purificateur de sang (10) est configuré de sorte que la valeur quadratique du coefficient de corrélation entre la différence de pression transmembranaire Pj, où j est un nombre entier non inférieur à 3, et une concentration de fuite de soluté Nj à S = 240 minutes comme instant 240 minutes après le début de la purification du sang n'est pas inférieure à 0,4.

**41.** Dispositif pour déterminer la différence de pression transmembranaire à travers la membrane de purification du sang (21) selon l'une quelconque des revendications 38 à 40, où le dispositif est configuré pour calculer, à partir d'une valeur mesurée réellement RNa(Tn) de la concentration de fuite de soluté sous la différence de pression transmembranaire Pa(Tn) à chacun des un ou plusieurs instants prédéterminés Tn après le début de la purification du sang, un rapport entre la concentration de fuite de soluté cible Na(Tn) et la valeur mesurée réellement à chacun des instants prédéterminés Tn, pour multiplier la différence de pression transmembranaire Pa(Tn) par le rapport pour obtenir une différence de pression transmembranaire Pa'(Tn), et pour déterminer la différence de pression transmembranaire Pa'(Tn) comme étant la différence de pression transmembranaire pendant la purification du sang.

**42.** Dispositif pour déterminer la différence de pression transmembranaire à travers la membrane de purification du sang (21) selon l'une quelconque des revendications 38 à 40, où le dispositif est configuré pour modifier la différence de pression transmembranaire Pa(Tn), tout en maintenant inchangée la valeur moyenne P des différences de pression transmembranaire pendant tout le traitement de purification du sang et empêchant le débit de fourniture du fluide de remplacement à chacun des instants prédéterminés Tn d'atteindre une limite supérieure, pour obtenir une différence de pression transmembranaire Pa"(Tn), et pour déterminer la différence de pression transmembranaire Pa"(Tn) comme étant la différence de pression transmembranaire pendant la purification du sang.

Fig. 1

Fig. 2

T1 □
T2 △
T3 ✕
T4 ✳
T5 ○

Fn(T1)

Fn(T2)

Fn(T3)

Fn(T4)

Fn(T5)

N [µg/mL]

P [mmHg]

# Fig. 3

15

| FUNCTION ACQUISITION MEANS | 90 |
| FUNCTION STORAGE MEANS | 91 |
| TRANSMEMBRANE PRESSURE DIFFERENCE CALCULATION MEANS | 92 |
| TRANSMEMBRANE PRESSURE DIFFERENCE CONTROL MEANS | 93 |

# Fig. 4

## Fig. 5

EP 3 659 641 B1

Fig. 6

# Fig. 7

# Fig. 8

Fig. 9

EP 3 659 641 B1

# Fig. 10

# Fig. 11

## Fig. 12

Fig. 13

# Fig. 14

Fig. 15

## Fig. 16

# Fig. 17

TRANSMEMBRANE PRESSURE DIFFERENCE[mmHg]

REPLACEMENT FLUID FLOW RATE

REPLACEMENT FLUID FLOW RATE[mL/min]

SET TRANSMEMBRANE PRESSURE DIFFERENCE

Tn

# Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001112863 A
- WO 2013141309 A
- JP 6070348 B